# EUROPEAN PATENT APPLICATION

(11) **EP 1 787 995 A1**
(43) Date of publication of application: **23.05.2007**
(21) Application number: 05025131.3
(22) Date of filing: 17.11.2005
(51) Int. Cl.: C07K 14/435

(54) **Recombinant mussel byssus protein**

(71) Applicant: Technische Universität München, 80333 München (DE)
(72) Inventor: Scheibel, Thomas, 81673 München (DE)
(74) Representative: Reinhard - Skuhra - Weise & Partner

(57) **Abstract**

The present invention is directed to a recombinant mussel byssus protein, an isolated nucleic acid encoding same and a host cell, which has been transformed with an expression vector containing said nucleic acid. The present invention is further directed to a kit of parts or a co-expression system for a mussel byssus protein and to a method of producing said recombinant mussel byssus protein. Furthermore, the invention pertains to a method of producing threads from said proteins as well as threads obtainable therefrom and their use in various fields of technology and medicine.

### Annex to the application documents - subsequently filed sequences listing

SEQUENCE LISTING

<110>  Technische Universitaet Muenchen

<120>  Recombinant mussel byssus protein

<130>  P19578

<160>  10

<170>  PatentIn version 3.3

<210>  1
<211>  2703
<212>  DNA
<213>  Mytilus edulis

<400>  1
atggttcggt tctccctagc atcggtacta ttactggcag tcaccagcac agctttcgct      60

ggaccagtta gtgattatgg tggtggtgga atcaaagtag taccctacca cggaggcgga     120

ggtggaagcg gcggcggtgg cggtggaggc catggcggaa gcggtattgg tggtatcgga     180

ggaggatcat cacatgcaca tgcccactct tcagcatctg cccatgtgca ccattttgga     240

ccaggtggat cttcacatgc atcagctggt tcatcatccc atgcatccgc atcccataac     300

ggtttaggag gtggcagtgc tcatgcacat agcagttcca gcgccaacgc tcattccggt     360

ggattcggtg gattcggcgg tattggtggt attggcggta ttggcccagg aggaagtgtc     420

ggaggcggta ttggcccagg aggaagtgtc ggaggcggca ttggcggtat tggcggtatt     480

ggcggcggtg gtggaccagg cggtaatggc ggtatcggat tcggaccagg attcggagga     540

ggattcggac caggttcatc tgctagtgga tccggaagtg gcagcgcatt cggtggtcca     600

ggaggttcaa gcgcaagcgc aaacgcagct gcacgtgcaa atgcaaatgg tggtggagga     660

ttcggtggac caggtacccc aggaaactca ggaccaccag gccaacccgg actaccagga     720

gcaccaggcc aaccaggacg tccaggaagt accccaccag gtcgaccagg aaaccccgga     780

ccaccaggtc aaccaggtaa cccaggacgt ccaggctctt caggaagacc aggaggatcc     840

ggccaaccag gaggtccagg acgtccagga acccccggca aaccaggaaa ccgaggacaa     900

ccaggacagc caggcggccc aggacaacca ggtcacccag gagcaggagg acaaccagga     960

cgaaacggaa atccaggaaa ccccggtaaa ccaggaacac caggtcaccc aggaacagca    1020

ggatcacgag gaatgccagg aaccccagga accccaggac aaccaggaat tccaggcacc    1080

gtcggaggac gaggaccaag aggaccagct ggaatcatcg gattaattgg accaaaagga    1140

aatccaggag agccaggaaa tccaggtgca ccaggaggcc caggatctac aggaccacaa    1200

ggaccacaag gaccagccgg aggaccagga gcatcaggcg gaccaggaga caaaggcgca    1260

ccaggtacac caggaggaac tggaccaaga ggaccaatcg gaccatcagg accatcagga    1320

gcaccagggg accaaggacc acaaggaggt agaggaacac caggactcgc aggcaaacca    1380

ggacctaaag gactacaagg atcaaatgga gaagttggac cccaaggacc atctggaccc 1440

gcaggaccac aaggcccaca aggaaagaac ggtgtcaaag gagcagcagg agatcaagga 1500

gctaggggac cagaaggaaa agccggacca gctggaccac aaggagaaac aggaccaaaa 1560

ggaccaacag gagcacaagg accagccggt ccagccggac catcaggaga acaaggacca 1620

ggaggggaaa gaggaggcca gggaccacaa ggagctgaag gaccaagtgg accagcagga 1680

ccaagaggac cagcaggatc acaaggacca agtggtgaac gcggagaacc aggagcacca 1740

ggtaaaaaag gaccaaatgg agaccgagga aaccaaggat caccaggagc accaggcaaa 1800

aacggagcac gaggaaatag aggatcaaga ggaagcaacg gatcacccgg cagatcagga 1860

tcaccaggaa gccgaggaaa accaggacca caaggaccac atggaccaag aggagcaaga 1920

ggatcaccag gacaaaaagg accacgtgga gaccaaggag caccaggtgt tattcgtatt 1980

gttatcgatg accagagaac aggaccagaa gttgcagaat tcccaggatt tggtggattc 2040

ggaggagctt cagctaacgc agcaagttca gcaaatgcat ttgctggtgg acccggtggt 2100

tccgctggag caggttcatc atcaggagct aacgcaaacg caggtggatt cccattcgga 2160

ggaggaccat tcggaggagc aggaggtggt cccggagcag caggaggccc aggaggagca 2220

ggaggcccag gaggagtagg aggaggagtt ggaggtggac caggaggagt aggaggtgga 2280

gtaggaggtg gaccaggagg agtaggaggt ggaccaggag gagcaggacc aggaggagca 2340

ggaggatttg gaccaggagg agcaggagga tttggtggat ttggaggagg atctagcgct 2400

ggagcatcat catcaggatc agcatctgca tctaacggtg gaccattcgg agtactcaat 2460

gtaggacccg gaggtagaat cggtggtgga agcgcatcag catctgcagc atctagagca 2520

catgcacacg cttttggtgg tctcggaggg ggaagtgcct cagctggtag tcattcctca 2580

tctagctcac actcatttgg cggacacgta ttccacagtg tgacccatca tggaggtcca 2640

tcacatgttt caagcggagg tcacggaggt catggaggag gtccatacaa acctggatat 2700

taa 2703

<210> 2
<211> 2025
<212> DNA
<213> Mytilus edulis

<400> 2
atggtctaca aactcctgac cgtgtgtctt gtagcatctc ttctagagat ttgcttagct 60

gactataacg gcaacaaaca gtatggcggc agatacggca acagatacgg aaacggttta 120

ggaggcggta atggtggtgc aggagccgta gcccatgccc atgcccatgc ccatgccagt 180

gccggagcaa acggaagagc aagagcacat gcacgagcct tggcccatgc acatgccggt 240

ggtggcgctg cacatggaca cccaggattc ccagttggtg gtagcgcaag cgcagccgca 300

cgagcagcag cacgagcatc agcaggagga ttaggtggat tcggatcagc agcagccaat 360

gcagcagcag cagcaagagc aggagcagga tttggtggat tcggtggatt aggaggattc 420

ggaggactcg gaggagttgg cggtccaggt caaccaggac atgccggtaa acacggaacc 480

gcaggagcag caggcaaagc aggacgtcca ggaccatgtg gagatagagg ggcaccagga 540

gtaccaggca aacaaggacc agtaggagga caaggaccag caggaccacg aggaccacga 600

ggagatgaag gaccagttgg accaaagggc gaaccaggag caagaggagc tgatggtaaa 660

ccaggagaca aaggacctga tggagaaacc ggaccacaag gaccagctgg accaaaggga 720

caagtaggag accaaggcaa accaggagca aagggagaaa ccggagatca aggagcacga 780

ggtgaagcag gaaaggccgg cgaacaagga ccaggaggca tccaaggacc aaaggaccca 840

gtaggaggac aaggaccagc aggaccagcc ggaccactcg gaccacaagg accaatgggt 900

gaacgaggac cacaaggacc aacaggatca gaaggaccag ttggagcacc aggaccaaag 960

ggatcagtcg gagaccaagg agcacaagga gaccaaggag caactggcgc tgatggcaaa 1020

aagggagaac caggagagag aggacaacaa ggagcagcag gaccagtcgg ccgaccagga 1080

ccaagaggag atagaggagc aaagggaatt caaggaagcc gaggacgacc aggtggtatg 1140

ggtagacgag gaaaccgtgg atcccaagga gcagtaggac cacgaggaga aactggccca 1200

gacggtaacc aaggacaacg tggagaacaa ggagcaccag gagttatcac ccttgtcatt 1260

gaagacctca gaacagccgg agtagaaagc cccgtagaaa cctttgacgc aggagcagga 1320

accggtggac cagcaccagg agtaggagca gcagcaacag caggagcatt tgcaggagca 1380

ggaccaggag gagctaatgc aggaggaaac gcagccgcag gagcaggacc aggagtagga 1440

ccaggaggac tcggaggact aggaggactt ggtgcaggtg gactcggagg tggactcggc 1500

ggtggactcg gaggattagg aggagcagga ggtttaggtg gtggactcgg aggattagga 1560

ggaggtttag gtggtggact cggaggttta ggaggtggag caggaggagc aggaggcgca 1620

ggagcaggag gaaacggtgg agcaggagca ggaggagcag gaggaaacgg tggaggatca 1680

gccgcagcac gagcagcagc acaagcagca gcagcagcag gaggaaacgg tggagcagca 1740

caagcagcag cacaagcagc agcatcagca gcagcaaatt caggacttgg agcaggagca 1800

gcaagagcag cagcatcagc agccgctaga gcaaccgtag caggacatgg aagtggaacc 1860

gccgcagcag cagccaacgc agccgcacaa gcacatgcag caacacgagg acaaggagga 1920

tcacacgcac acgctgccgc cgcagctcac gcagccgcaa gtagcgtaat ccatggtggt 1980

gactatcacg gaaacgatgc cggctatcac aaaccaggat attaa 2025

<210> 3
<211> 900
<212> PRT

<213>  Mytilus edulis

<400>  3
   Met Val Arg Phe Ser Leu Ala Ser Val Leu Leu Leu Ala Val Thr Ser
   1               5                   10                  15
   Thr Ala Phe Ala Gly Pro Val Ser Asp Tyr Gly Gly Gly Gly Ile Lys
               20                  25                  30
   Val Val Pro Tyr His Gly Gly Gly Gly Gly Ser Gly Gly Gly Gly Gly
           35                  40                  45
   Gly Gly His Gly Gly Ser Gly Ile Gly Gly Ile Gly Gly Gly Ser Ser
       50                  55                  60
   His Ala His Ala His Ser Ser Ala Ser Ala His Val His His Phe Gly
   65                  70                  75                  80
   Pro Gly Gly Ser Ser His Ala Ser Ala Gly Ser Ser Ser His Ala Ser
                   85                  90                  95
   Ala Ser His Asn Gly Leu Gly Gly Gly Ser Ala His Ala His Ser Ser
               100                 105                 110
   Ser Ser Ala Asn Ala His Ser Gly Gly Phe Gly Gly Phe Gly Gly Ile
           115                 120                 125
   Gly Gly Ile Gly Gly Ile Gly Pro Gly Gly Ser Val Gly Gly Gly Ile
       130                 135                 140
   Gly Pro Gly Gly Ser Val Gly Gly Gly Ile Gly Gly Ile Gly Gly Ile
   145                 150                 155                 160
   Gly Gly Gly Gly Gly Pro Gly Gly Asn Gly Gly Ile Gly Phe Gly Pro
                   165                 170                 175
   Gly Phe Gly Gly Gly Phe Gly Pro Gly Ser Ser Ala Ser Gly Ser Gly
               180                 185                 190
   Ser Gly Ser Ala Phe Gly Gly Pro Gly Gly Ser Ser Ala Ser Ala Asn
           195                 200                 205
   Ala Ala Ala Arg Ala Asn Ala Asn Gly Gly Gly Gly Phe Gly Gly Pro
       210                 215                 220
   Gly Thr Pro Gly Asn Ser Gly Pro Pro Gly Gln Pro Gly Leu Pro Gly
   225                 230                 235                 240
   Ala Pro Gly Gln Pro Gly Arg Pro Gly Ser Thr Pro Pro Gly Arg Pro
                   245                 250                 255
   Gly Asn Pro Gly Pro Pro Gly Gln Pro Gly Asn Pro Gly Arg Pro Gly
               260                 265                 270
   Ser Ser Gly Arg Pro Gly Gly Ser Gly Gln Pro Gly Gly Pro Gly Arg
           275                 280                 285
   Pro Gly Thr Pro Gly Lys Pro Gly Asn Arg Gly Gln Pro Gly Gln Pro
       290                 295                 300
   Gly Gly Pro Gly Gln Pro Gly His Pro Gly Ala Gly Gly Gln Pro Gly
   305                 310                 315                 320
   Arg Asn Gly Asn Pro Gly Asn Pro Gly Lys Pro Gly Thr Pro Gly His
                   325                 330                 335
   Pro Gly Thr Ala Gly Ser Arg Gly Met Pro Gly Thr Pro Gly Thr Pro
               340                 345                 350
   Gly Gln Pro Gly Ile Pro Gly Thr Val Gly Gly Arg Gly Pro Arg Gly
           355                 360                 365
   Pro Ala Gly Ile Ile Gly Leu Ile Gly Pro Lys Gly Asn Pro Gly Glu
       370                 375                 380
   Pro Gly Asn Pro Gly Ala Pro Gly Gly Pro Gly Ser Thr Gly Pro Gln
   385                 390                 395                 400
   Gly Pro Gln Gly Pro Ala Gly Gly Pro Gly Ala Ser Gly Gly Pro Gly
                   405                 410                 415
   Asp Lys Gly Ala Pro Gly Thr Pro Gly Gly Thr Gly Pro Arg Gly Pro
               420                 425                 430
   Ile Gly Pro Ser Gly Pro Ser Gly Ala Pro Gly Asp Gln Gly Pro Gln
           435                 440                 445
   Gly Gly Arg Gly Thr Pro Gly Leu Ala Gly Lys Pro Gly Pro Lys Gly
       450                 455                 460
   Leu Gln Gly Ser Asn Gly Glu Val Gly Pro Gln Gly Pro Ser Gly Pro
   465                 470                 475                 480
   Ala Gly Pro Gln Gly Pro Gln Gly Lys Asn Gly Val Lys Gly Ala Ala
                   485                 490                 495
   Gly Asp Gln Gly Ala Arg Gly Pro Glu Gly Lys Ala Gly Pro Ala Gly
   500 505 510
   Pro Gln Gly Glu Thr Gly Pro Lys Gly Pro Thr Gly Ala Gln Gly Pro
   515 520 525
   Ala Gly Pro Ala Gly Pro Ser Gly Glu Gln Gly Pro Gly Gly Glu Arg
   530 535 540
   Gly Gly Gln Gly Pro Gln Gly Ala Glu Gly Pro Ser Gly Pro Ala Gly
   545 550 555 560
   Pro Arg Gly Pro Ala Gly Ser Gln Gly Pro Ser Gly Glu Arg Gly Glu
   565 570 575
   Pro Gly Ala Pro Gly Lys Lys Gly Pro Asn Gly Asp Arg Gly Asn Gln
   580 585 590
   Gly Ser Pro Gly Ala Pro Gly Lys Asn Gly Ala Arg Gly Asn Arg Gly
   595 600 605
   Ser Arg Gly Ser Asn Gly Ser Pro Gly Arg Ser Gly Ser Pro Gly Ser
   610 615 620
   Arg Gly Lys Pro Gly Pro Gln Gly Pro His Gly Pro Arg Gly Ala Arg
   625 630 635 640
   Gly Ser Pro Gly Gln Lys Gly Pro Arg Gly Asp Gln Gly Ala Pro Gly
   645 650 655
   Val Ile Arg Ile Val Ile Asp Asp Gln Arg Thr Gly Pro Glu Val Ala
   660 665 670
   Glu Phe Pro Gly Phe Gly Gly Phe Gly Gly Ala Ser Ala Asn Ala Ala
   675 680 685
   Ser Ser Ala Asn Ala Phe Ala Gly Gly Pro Gly Gly Ser Ala Gly Ala
   690 695 700
   Gly Ser Ser Ser Gly Ala Asn Ala Asn Ala Gly Gly Phe Pro Phe Gly
   705 710 715 720
   Gly Gly Pro Phe Gly Gly Ala Gly Gly Gly Pro Gly Ala Ala Gly Gly
   725 730 735
   Pro Gly Gly Ala Gly Gly Pro Gly Gly Val Gly Gly Gly Val Gly Gly
   740                 745                 750
   Gly Pro Gly Gly Val Gly Gly Gly Val Gly Gly Gly Pro Gly Gly Val
           755                 760                 765
   Gly Gly Gly Pro Gly Gly Ala Gly Pro Gly Gly Ala Gly Gly Phe Gly
       770                 775                 780
   Pro Gly Gly Ala Gly Gly Phe Gly Gly Phe Gly Gly Gly Ser Ser Ala
   785                 790                 795                 800
   Gly Ala Ser Ser Ser Gly Ser Ala Ser Ala Ser Asn Gly Gly Pro Phe
                   805                 810                 815
   Gly Val Leu Asn Val Gly Pro Gly Gly Arg Ile Gly Gly Gly Ser Ala
               820                 825                 830
   Ser Ala Ser Ala Ala Ser Arg Ala His Ala His Ala Phe Gly Gly Leu
           835                 840                 845
   Gly Gly Gly Ser Ala Ser Ala Gly Ser His Ser Ser Ser Ser Ser His
       850                 855                 860
   Ser Phe Gly Gly His Val Phe His Ser Val Thr His His Gly Gly Pro
   865                 870                 875                 880
   Ser His Val Ser Ser Gly Gly His Gly Gly His Gly Gly Gly Pro Tyr
                   885                 890                 895
   Lys Pro Gly Tyr
               900

<210> 4
<211> 674
<212> PRT
<213> Mytilus edulis

<400> 4

Met Val Tyr Lys Leu Leu Thr Val Cys Leu Val Ala Ser Leu Leu Glu
1               5                   10                  15

Ile Cys Leu Ala Asp Tyr Asn Gly Asn Lys Gln Tyr Gly Gly Arg Tyr
            20                  25                  30

Gly Asn Arg Tyr Gly Asn Gly Leu Gly Gly Gly Asn Gly Gly Ala Gly
        35                  40                  45

Ala Val Ala His Ala His Ala His Ala His Ala Ser Ala Gly Ala Asn

50                  55                  60

Gly Arg Ala Arg Ala His Ala Arg Ala Leu Ala His Ala His Ala Gly
65                  70                  75                  80

Gly Gly Ala Ala His Gly His Pro Gly Phe Pro Val Gly Gly Ser Ala
                85                  90                  95

Ser Ala Ala Ala Arg Ala Ala Ala Arg Ala Ser Ala Gly Gly Leu Gly
            100                 105                 110

Gly Phe Gly Ser Ala Ala Ala Asn Ala Ala Ala Ala Ala Arg Ala Gly
        115                 120                 125

Ala Gly Phe Gly Gly Phe Gly Gly Leu Gly Gly Phe Gly Gly Leu Gly
    130                 135                 140

Gly Val Gly Gly Pro Gly Gln Pro Gly His Ala Gly Lys His Gly Thr
145                 150                 155                 160

Ala Gly Ala Ala Gly Lys Ala Gly Arg Pro Gly Pro Cys Gly Asp Arg
                165                 170                 175

Gly Ala Pro Gly Val Pro Gly Lys Gln Gly Pro Val Gly Gly Gln Gly
            180                 185                 190

Pro Ala Gly Pro Arg Gly Pro Arg Gly Asp Glu Gly Pro Val Gly Pro
        195                 200                 205

Lys Gly Glu Pro Gly Ala Arg Gly Ala Asp Gly Lys Pro Gly Asp Lys
    210                 215                 220

Gly Pro Asp Gly Glu Thr Gly Pro Gln Gly Pro Ala Gly Pro Lys Gly
225                 230                 235                 240

Gln Val Gly Asp Gln Gly Lys Pro Gly Ala Lys Gly Glu Thr Gly Asp
                245                 250                 255

Gln Gly Ala Arg Gly Glu Ala Gly Lys Ala Gly Glu Gln Gly Pro Gly
            260                 265                 270

Gly Ile Gln Gly Pro Lys Gly Pro Val Gly Gly Gln Gly Pro Ala Gly
        275                 280                 285

Pro Ala Gly Pro Leu Gly Pro Gln Gly Pro Met Gly Glu Arg Gly Pro
    290                 295                 300

Gln Gly Pro Thr Gly Ser Glu Gly Pro Val Gly Ala Pro Gly Pro Lys
305                 310                 315                 320

Gly Ser Val Gly Asp Gln Gly Ala Gln Gly Asp Gln Gly Ala Thr Gly
                325                 330                 335

Ala Asp Gly Lys Lys Gly Glu Pro Gly Glu Arg Gly Gln Gln Gly Ala
            340                 345                 350

Ala Gly Pro Val Gly Arg Pro Gly Pro Arg Gly Asp Arg Gly Ala Lys
        355                 360                 365

Gly Ile Gln Gly Ser Arg Gly Arg Pro Gly Gly Met Gly Arg Arg Gly
    370                 375                 380

Asn Arg Gly Ser Gln Gly Ala Val Gly Pro Arg Gly Glu Thr Gly Pro
385                 390                 395                 400

Asp Gly Asn Gln Gly Gln Arg Gly Glu Gln Gly Ala Pro Gly Val Ile
                405                 410                 415

Thr Leu Val Ile Glu Asp Leu Arg Thr Ala Gly Val Glu Ser Pro Val
            420                 425                 430

Glu Thr Phe Asp Ala Gly Ala Gly Thr Gly Gly Pro Ala Pro Gly Val
        435                 440                 445

Gly Ala Ala Ala Thr Ala Gly Ala Phe Ala Gly Ala Gly Pro Gly Gly
    450                 455                 460

Ala Asn Ala Gly Gly Asn Ala Ala Ala Gly Ala Gly Pro Gly Val Gly
465                 470                 475                 480

Pro Gly Gly Leu Gly Gly Leu Gly Gly Leu Gly Ala Gly Gly Leu Gly
                485                 490                 495

Gly Gly Leu Gly Gly Gly Leu Gly Gly Leu Gly Gly Ala Gly Gly Leu
            500                 505                 510

Gly Gly Gly Leu Gly Gly Leu Gly Gly Gly Leu Gly Gly Gly Leu Gly
        515                 520                 525

Gly Leu Gly Gly Gly Ala Gly Gly Ala Gly Gly Ala Gly Ala Gly Gly
    530                 535                 540

Asn Gly Gly Ala Gly Ala Gly Gly Ala Gly Gly Asn Gly Gly Gly Ser
545                 550                 555                 560

Ala Ala Ala Arg Ala Ala Ala Gln Ala Ala Ala Ala Ala Gly Gly Asn
                565                 570                 575

Gly Gly Ala Ala Gln Ala Ala Ala Gln Ala Ala Ala Ser Ala Ala Ala
            580                 585                 590

Asn Ser Gly Leu Gly Ala Gly Ala Ala Arg Ala Ala Ala Ser Ala Ala
        595                 600                 605

Ala Arg Ala Thr Val Ala Gly His Gly Ser Gly Thr Ala Ala Ala Ala
    610                 615                 620

Ala Asn Ala Ala Ala Gln Ala His Ala Ala Thr Arg Gly Gln Gly Gly
625                 630                 635                 640

Ser His Ala His Ala Ala Ala Ala Ala His Ala Ala Ala Ser Ser Val
                645                 650                 655

Ile His Gly Gly Asp Tyr His Gly Asn Asp Ala Gly Tyr His Lys Pro
            660                 665                 670

Gly Tyr

<210>  5
<211>  536
<212>  PRT
<213>  Saccharomyces cerevisiae

<400>  5

Met Arg Phe Pro Ser Ile Phe Thr Ala Val Leu Phe Ala Ala Ser Ser
1               5                   10                  15

Ala Leu Ala His Pro Gly Phe Phe Thr Ser Ile Gly Gln Met Thr Asp
            20                  25                  30

Leu Ile His Thr Glu Lys Asp Leu Val Thr Ser Leu Lys Asp Tyr Ile
        35                  40                  45

Lys Ala Glu Glu Asp Lys Leu Glu Gln Ile Lys Lys Trp Ala Glu Lys
    50                  55                  60

Leu Asp Arg Leu Thr Ser Thr Ala Thr Lys Asp Pro Glu Gly Phe Val
65                  70                  75                  80

Gly His Pro Val Asn Ala Phe Lys Leu Met Lys Arg Leu Asn Thr Glu
                85                  90                  95

Trp Ser Glu Leu Glu Asn Leu Val Leu Lys Asp Met Ser Asp Gly Phe
            100                 105                 110

Ile Ser Asn Leu Thr Ile Gln Arg Pro Val Leu Ser Asn Asp Glu Asp
        115                 120                 125

Gln Val Gly Ala Ala Lys Ala Leu Leu Arg Leu Gln Asp Thr Tyr Asn
    130                 135                 140

Leu Asp Thr Asp Thr Ile Ser Lys Gly Asn Leu Pro Gly Val Lys His
145                 150                 155                 160

Lys Ser Phe Leu Thr Ala Glu Asp Cys Phe Glu Leu Gly Lys Val Ala
                165                 170                 175

Tyr Thr Glu Ala Asp Tyr Tyr His Thr Glu Leu Trp Met Glu Gln Ala
            180                 185                 190

Leu Arg Gln Leu Asp Glu Gly Glu Ile Ser Thr Ile Asp Lys Val Ser
        195                 200                 205

Val Leu Asp Tyr Leu Ser Tyr Ala Val Tyr Gln Gln Gly Asp Leu Asp
    210                 215                 220

Lys Ala Leu Leu Leu Thr Lys Lys Leu Leu Glu Leu Asp Pro Glu His
225                 230                 235                 240

Gln Arg Ala Asn Gly Asn Leu Lys Tyr Phe Glu Tyr Ile Met Ala Lys
                245                 250                 255

Glu Lys Asp Val Asn Lys Ser Ala Ser Asp Asp Gln Ser Asp Gln Lys
            260                 265                 270

Thr Thr Pro Lys Lys Lys Gly Val Ala Val Asp Tyr Leu Pro Glu Arg
        275                 280                 285

Gln Lys Tyr Glu Met Leu Cys Arg Gly Glu Gly Ile Lys Met Thr Pro
    290                 295                 300

Arg Arg Gln Lys Lys Leu Phe Cys Arg Tyr His Asp Gly Asn Arg Asn
305                 310                 315                 320

Pro Lys Phe Ile Leu Ala Pro Ala Lys Gln Glu Asp Glu Trp Asp Lys
                325                 330                 335

Pro Arg Ile Ile Arg Phe His Asp Ile Ile Ser Asp Ala Glu Ile Glu
            340                 345                 350

Ile Val Lys Asp Leu Ala Lys Pro Arg Leu Ser Arg Ala Thr Val His
        355                 360                 365

Asp Pro Glu Thr Gly Lys Leu Thr Thr Ala Gln Tyr Arg Val Ser Lys
    370                 375                 380

Ser Ala Trp Leu Ser Gly Tyr Glu Asn Pro Val Val Ser Arg Ile Asn
385                 390                 395                 400

Met Arg Ile Gln Asp Leu Thr Gly Leu Asp Val Ser Thr Ala Glu Glu
                405                 410                 415

Leu Gln Val Ala Asn Tyr Gly Val Gly Gly Gln Tyr Glu Pro His Phe
            420                 425                 430

Asp Phe Ala Arg Lys Asp Glu Pro Asp Ala Phe Lys Glu Leu Gly Thr
        435                 440                 445

Gly Asn Arg Ile Ala Thr Trp Leu Phe Tyr Met Ser Asp Val Ser Ala
    450                 455                 460

Gly Gly Ala Thr Val Phe Pro Glu Val Gly Ala Ser Val Trp Pro Lys
465                 470                 475                 480

Lys Gly Thr Ala Val Phe Trp Tyr Asn Leu Phe Ala Ser Gly Glu Gly
                485                 490                 495

Asp Tyr Ser Thr Arg His Ala Ala Cys Pro Val Leu Val Gly Asn Lys
            500                 505                 510

Trp Val Ser Asn Lys Trp Leu His Glu Arg Gly Gln Glu Phe Arg Arg
        515                 520                 525

Pro Cys Thr Leu Ser Glu Leu Glu
    530                 535

<210> 6
<211> 510
<212> PRT
<213> Saccharomyces cerevisiae

<400> 6

Met Arg Phe Pro Ser Ile Phe Thr Ala Val Leu Phe Ala Ala Ser Ser
1               5                   10                  15

Ala Leu Ala Asp Ala Pro Glu Glu Glu Asp His Val Leu Val Leu Arg
            20                  25                  30

Lys Ser Asn Phe Ala Glu Ala Leu Ala Ala His Lys Tyr Leu Leu Val
        35                  40                  45

Glu Phe Tyr Ala Pro Trp Cys Gly His Cys Lys Ala Leu Ala Pro Glu
    50                  55                  60

Tyr Ala Lys Ala Ala Gly Lys Leu Lys Ala Glu Gly Ser Glu Ile Arg
65                  70                  75                  80

Leu Ala Lys Val Asp Ala Thr Glu Glu Ser Asp Leu Ala Gln Gln Tyr
                85                  90                  95

Gly Val Arg Gly Tyr Pro Thr Ile Lys Phe Phe Arg Asn Gly Asp Thr
            100                 105                 110

Ala Ser Pro Lys Glu Tyr Thr Ala Gly Arg Glu Ala Asp Asp Ile Val
        115                 120                 125

Asn Trp Leu Lys Lys Arg Thr Gly Pro Ala Ala Thr Thr Leu Pro Asp
    130                 135                 140

Gly Ala Ala Ala Glu Ser Leu Val Glu Ser Ser Glu Val Ala Val Ile
145                 150                 155                 160

Gly Phe Phe Lys Asp Val Glu Ser Asp Ser Ala Lys Gln Phe Leu Gln
                165                 170                 175

Ala Ala Glu Ala Ile Asp Asp Ile Pro Phe Gly Ile Thr Ser Asn Ser
            180                 185                 190

Asp Val Phe Ser Lys Tyr Gln Leu Asp Lys Asp Gly Val Val Leu Phe
        195                 200                 205

Lys Lys Phe Asp Glu Gly Arg Asn Asn Phe Glu Gly Glu Val Thr Lys
    210                 215                 220

Glu Asn Leu Leu Asp Phe Ile Lys His Asn Gln Leu Pro Leu Val Ile
225                 230                 235                 240

Glu Phe Thr Glu Gln Thr Ala Pro Lys Ile Phe Gly Gly Glu Ile Lys
                245                 250                 255

Thr His Ile Leu Leu Phe Leu Pro Lys Ser Val Ser Asp Tyr Asp Gly
            260                 265                 270

Lys Leu Ser Asn Phe Lys Thr Ala Ala Glu Ser Phe Lys Gly Lys Ile

275                 280                 285

Leu Phe Ile Phe Ile Asp Ser Asp His Thr Asp Asn Gln Arg Ile Leu
    290                 295                 300

Glu Phe Phe Gly Leu Lys Lys Glu Glu Cys Pro Ala Val Arg Leu Ile
305                 310                 315                 320

Thr Leu Glu Glu Glu Met Thr Lys Tyr Lys Pro Glu Ser Glu Glu Leu
                325                 330                 335

Thr Ala Glu Arg Ile Thr Glu Phe Cys His Arg Phe Leu Glu Gly Lys
            340                 345                 350

Ile Lys Pro His Leu Met Ser Gln Glu Leu Pro Glu Asp Trp Asp Lys
        355                 360                 365

Gln Pro Val Lys Val Leu Val Gly Lys Asn Phe Glu Asp Val Ala Phe
    370                 375                 380

Asp Glu Lys Lys Asn Val Phe Val Glu Phe Tyr Ala Pro Trp Cys Gly
385                 390                 395                 400

His Cys Lys Gln Leu Ala Pro Ile Trp Asp Lys Leu Gly Glu Thr Tyr
                405                 410                 415

Lys Asp His Glu Asn Ile Val Ile Ala Lys Met Asp Ser Thr Ala Asn
            420                 425                 430

Glu Val Glu Ala Val Lys Val His Ser Phe Pro Thr Leu Lys Phe Phe
        435                 440                 445

Pro Ala Ser Ala Asp Arg Thr Val Ile Asp Tyr Asn Gly Glu Arg Thr
    450                 455                 460

Leu Asp Gly Phe Lys Lys Phe Leu Glu Ser Gly Gly Gln Asp Gly Ala
465                 470                 475                 480

Gly Asp Asp Asp Asp Leu Glu Asp Leu Glu Glu Ala Glu Glu Pro Asp
                485                 490                 495

Met Glu Glu Asp Asp Asp Gln Lys Ala Val Lys Asp Glu Leu
            500                 505                 510

<210>  7
<211>  3876
<212>  DNA
<213>  Mytilus edulis

<400> 7
ccgcggtcat tacagttcat ctttcacagc tttctgatca tcgtcttcct ccatgtctgg 60
ctcctctgct tcttccaggt cctcgagatc gtcatcatcc cctgccccat cctggccacc 120
cgagaggtcc ttaaagaatt ttggtaggtc gcacgcaagg ggcaacatta gttactggca 180
cctgtcggca ctggcaggaa agaacttgag tgtggggaag ctgtgcactt tgacggcctc 240
cacctcgttg gcagtcgagt ccatcttggc gatgacgatg ttctcatggt ccttgtacgt 300
ctctcccagt ttatcccaaa tgggagccaa ctgtttgcag tgaccacacc atggggcata 360
gaactccaca aagacgtttt ttttctcatc aaaagccacg tcttcaaagt tcttcccaac 420
aagcaccttg acaggctgct tgtcccagtc ctccggcagc tcctggctca tcaggtgggg 480
cttgattttg ccctccagga agcggtggca gaactctgtg atcctctctg ccgtcagctc 540
ctccgattcg ggcttgtact tggtcatctc ctcctccagg gtgatgaggc gcacggccgg 600
gcactcttcc ttcttcaggc caaagaactc gaggatgcgc tggttgtcgg tgtggtcgct 660
gtcgatgaag atgaacagga tcttgccctt gaagctctcg gctgctgttt tgaagttgct 720
cagtttgccg tcatagtcag acacactctt gggcaagaac agcaggatgt gagtcttgat 780
ttcacctcca aaaatcttcg gggctgtctg ctcggtgaac tcgatgacaa ggggcagctg 840
gttgtgtttg ataaagtcca gcaggttctc cttggtgacc tccccttcaa agttgttccg 900
gccttcatca aacttcttaa agaggacaac cccatctttg tcgagctggt atttggagaa 960
cacgtcactg ttggaagtga tcccaaatgg tatgtcatcg atggcctctg ctgcctgcaa 1020
aaactgcttg gcagagtccg actccacgtc cttgaagaag ccgatgacag ccacctcgct 1080
ggactccacc aaggactctg cagctgcgcc gtcaggcagg gtggtggcag ccgggcccgt 1140
gcgcttcttc agccagttca cgatgtcatc agcctctctg ccagctgtat attccttggg 1200
ggaagccgtg tctccattcc tgaagaactt gatggtggga tagccgcgca cgccgtactg 1260
ctgggccagg tcagactcct ccgtggcgtc caccttggcc aacctgatct cggaaccttc 1320
tgccttcagc ttcccagcgg ctttggcata ctcaggggcc agagccttgc agtggccaca 1380
ggttccccgt atcttgaggt ggtcgtccat gaacacccgg cggtcgcgga ggcgcttcaa 1440
gcttttccgc agcaccagga cgtggtcctc ctcctccgga gcgtcagcta atgcggagga 1500
tgctgcgaat aaaactgcag taaaaattga aggaaatctc atggatccgg ggttttttct 1560
ccttgacgtt aaagtataga ggtatattaa caattttttg ttgatacttt tattacattt 1620
gaataagaag taatacaaac cgaaaatgtt gaaagtatta gttaaagtgg ttatgcagtt 1680
tttgcattta tatatctgtt aatagatcaa aaatcatcgc ttcgctgatt aattacccca 1740
gaaataaggc taaaaaacta atcgcattat catcctatgg ttgttaattt gattcgttca 1800
tttgaaggtt tgtggggcca ggttactgcc aatttttcct cttcataacc ataaaagcta 1860

gtattgtaga atctttattg ttcggagcag tgcggcgcga ggcacatctg cgtttcagga 1920

acgcgaccgg tgaagacgag gacgcacgga ggagagtctt ccttcggagg gctgtcaccc 1980

gctcggcggc ttctaatccg tacttcaata tagcaatgag cagttaagcg tattactgaa 2040

agttccaaag agaaggtttt tttaggctaa gataatgggg ctctttacat ttccacaaca 2100

tataagtaag attagatatg gatatgtata tggatatgta tatggtggta atgccatgta 2160

atatgattat taaacttctt tgcgtccatc caaaaaaaaa gtaagaattt ttgaaaattc 2220

aaggaattcg atatcaagct tatcgatacc gtcgacatga gatttccttc aatttttact 2280

gcagttttat tcgcagcatc ctccgcgcta gctcatccag gcttttttac ttcaattggt 2340

cagatgactg atttgatcca tactgagaaa gatctggtga cttctctgaa agattatatt 2400

aaggcagaag aggacaagtt agaacaaata aaaaaatggg cagagaagtt agatcggcta 2460

actagtacag cgacaaaaga tccagaagga tttgttgggc atccagtaaa tgcattcaaa 2520

ttaatgaaac gtctgaatac tgagtggagt gagttggaga atctggtcct taaggatatg 2580

tcagatggct ttatctctaa cctaaccatt cagagaccag tactttctaa tgatgaagat 2640

caggttgggg cagccaaagc tctgttacgt ctccaggata cctacaattt ggatacagat 2700

accatctcaa agggtaatct tccaggagtg aaacacaaat cttttctaac ggctgaggac 2760

tgctttgagt tgggcaaagt ggcctataca gaagcagatt attaccatac ggaactgtgg 2820

atggaacaag ccctaaggca actggatgaa ggcgagattt ctaccataga taaagtctct 2880

gttctagatt atttgagcta tgcggtatat cagcagggag acctggataa ggcacttttg 2940

ctcacaaaga agcttcttga actagatcct gaacatcaga gagctaatgg taacttaaaa 3000

tattttgagt atataatggc taaagaaaaa gatgtcaata agtctgcttc agatgaccaa 3060

tctgatcaga aaactacacc aaagaaaaaa ggggttgctg tggattacct gccagagaga 3120

cagaagtacg aaatgctgtg ccgtggggag ggtatcaaaa tgacccctcg gagacagaaa 3180

aaactctttt gccgctacca tgatggaaac cgtaatccta aatttattct ggctccagct 3240

aaacaggagg atgaatggga caagcctcgt attattcgct tccatgatat tatttctgat 3300

gcagaaattg aaatcgtcaa agacctagca aaaccaaggc tgagccgagc tacagtacat 3360

gaccctgaga ctggaaaatt gaccacagca cagtacagag tatctaagag tgcctggctc 3420

tctggctatg aaaatcctgt ggtgtctcga attaatatga gaatacaaga tctaacagga 3480

ctagatgttt ccacagcaga ggaattacag gtagcaaatt atggagttgg aggacagtat 3540

gaaccccatt ttgactttgc acggaaagat gagccagatg ctttcaaaga gctggggaca 3600

ggaaatagaa ttgctacatg gctgttttat atgagtgatg tgtctgcagg aggagccact 3660

gtttttcctg aagttggagc tagtgtttgg cccaaaaaag gaactgctgt tttctggtat 3720

aatctgtttg ccagtggaga aggagattat agtacacggc atgcagcctg tccagtgcta 3780

gttggcaaca aatgggtatc caataaatgg ctccatgaac gtggacaaga atttcgaaga 3840

ccttgtacgt tgtcagaatt ggaatgatga gggccc 3876

<210> 8
<211> 922
<212> PRT
<213> Mytilus edulis

<220>
<221> misc_feature
<222> (307)..(307)
<223> Xaa can be any naturally occurring amino acid

<400> 8

Met Val Tyr Lys Leu Leu Thr Val Cys Leu Val Ala Ser Leu Leu Glu
1               5                   10                  15

Ile Cys Leu Ala Asp Tyr Asn Gly Asn Lys Gln Tyr Gly Gly Arg Tyr
            20                  25                  30

Gly Asn Arg Tyr Gly Asn Gly Leu Gly Gly Gly Asn Gly Gly Ala Gly
        35                  40                  45

Ala Val Ala His Ala His Ala His Ala His Ala Ser Ala Gly Ala Asn
    50                  55                  60

Gly Arg Ala Arg Ala His Ala Arg Ala Leu Ala His Ala His Ala Gly
65                  70                  75                  80

Gly Gly Ala Ala His Gly His Pro Gly Phe Pro Val Gly Gly Ser Ala
                85                  90                  95

Ser Ala Ala Ala Arg Ala Ala Ala Arg Ala Ser Ala Gly Gly Leu Gly
            100                 105                 110

Gly Phe Gly Ser Ala Ala Ala Asn Ala Ala Ala Ala Ala Arg Ala Gly
        115                 120                 125

Ala Gly Phe Gly Gly Phe Gly Gly Leu Gly Gly Phe Gly Gly Leu Gly
    130                 135                 140

Gly Val Gly Gly Pro Gly Gln Pro Gly Gly Pro Gly Gly Pro Gly Gly
145                 150                 155                 160

Pro Gly Gly Pro Gly Gly Pro Gly Met Pro Gly Gly Pro Gly Gly Pro
                165                 170                 175

Ser Gly Pro Gly Thr Gly Gly Pro Gly Gln Pro Gly Gly Pro Gly Gly
            180                 185                 190

Pro Gly Gly Pro Gly Gly Pro Gly Gly Pro Ser Met Pro Gly Gly Pro
        195                 200                 205

Gly Gly Pro Gly Gly Pro Gly Met Pro Gly Gly Pro Gly Gly Pro Gly
    210                 215                 220

Gly Pro Gly Gly Ala Gly Gly Ile Pro Gly Met Thr Gly Pro Ala Gly
225                 230                 235                 240

Pro Pro Gly Pro Ala Gly Pro Gln Gly Pro Glu Gly Glu Gln Gly Pro
                245                 250                 255

Arg Gly Arg Thr Pro Ala Gly Thr Pro Gly Pro Pro Gly Asn Pro Gly
            260                 265                 270

Glu Pro Gly Gln Gly Gly Ala Pro Gly Ala Pro Gly Ala Pro Gly His
        275                 280                 285

Ala Gly Lys His Gly Thr Ala Gly Ala Ala Gly Lys Ala Gly Arg Pro
    290                 295                 300

Gly Pro Xaa Gly Gln Ala Gly Ala Ser Gly Ser Ser Gly Gln His Gly
305                 310                 315                 320

Ala Ser Gly Ala Pro Gly Arg Pro Gly Asn Pro Gly Ser Thr Gly Arg
                325                 330                 335

Pro Gly Ala Thr Gly Asp Pro Gly Arg Pro Gly Ala Thr Gly Thr Thr
            340                 345                 350

Gly Arg Pro Gly Pro Ser Gly Ala Pro Gly Asn Pro Gly Ala Pro Gly
        355                 360                 365

Ala Leu Gly Ala Pro Gly Pro Arg Gly Ser Pro Gly Phe Val Gly Leu
    370                 375                 380

Pro Gly Pro Arg Gly Ser Pro Gly Glu Pro Gly Asn Gln Gly Pro Ile
385                 390                 395                 400

Gly Gly Pro Gly Tyr Pro Gly Pro Arg Gly Pro Gln Gly Pro Asp Gly
                405                 410                 415

Ala Met Gly Pro Gln Gly Pro Cys Gly Asp Arg Gly Ala Pro Gly Val
            420                 425                 430

Pro Gly Lys Gln Gly Pro Val Gly Gly Gln Gly Pro Ala Gly Pro Arg
        435                 440                 445

Gly Pro Arg Gly Asp Glu Gly Pro Val Gly Pro Lys Gly Glu Pro Gly
    450                 455                 460

Ala Arg Gly Ala Asp Gly Lys Pro Gly Asp Lys Gly Pro Asp Gly Glu
465                 470                 475                 480

Thr Gly Pro Gln Gly Pro Ala Gly Pro Lys Gly Gln Val Gly Asp Gln
                485                 490                 495

Gly Lys Pro Gly Ala Lys Gly Glu Thr Gly Asp Gln Gly Ala Arg Gly
            500                 505                 510

Glu Ala Gly Lys Ala Gly Glu Gln Gly Pro Gly Gly Ile Gln Gly Pro
        515                 520                 525

Lys Gly Pro Val Gly Gly Gln Gly Pro Ala Gly Pro Ala Gly Pro Leu
    530                 535                 540

Gly Pro Gln Gly Pro Met Gly Glu Arg Gly Pro Gln Gly Pro Thr Gly
545                 550                 555                 560

Ser Glu Gly Pro Val Gly Ala Pro Gly Pro Lys Gly Ser Val Gly Asp
                565                 570                 575

Gln Gly Ala Gln Gly Asp Gln Gly Ala Thr Gly Ala Asp Gly Lys Lys
            580                 585                 590

Gly Glu Pro Gly Glu Arg Gly Gln Gln Gly Ala Ala Gly Pro Val Gly
        595                 600                 605

Arg Pro Gly Pro Arg Gly Asp Arg Gly Ala Lys Gly Ile Gln Gly Ser
    610                 615                 620

Arg Gly Arg Pro Gly Gly Met Gly Arg Arg Gly Asn Arg Gly Ser Gln
625                 630                 635                 640

Gly Ala Val Gly Pro Arg Gly Glu Thr Gly Pro Asp Gly Asn Gln Gly
                645                 650                 655

Gln Arg Gly Glu Gln Gly Ala Pro Gly Val Ile Thr Leu Val Ile Glu
            660                 665                 670

Asp Leu Arg Thr Ala Gly Val Glu Ser Pro Val Glu Thr Phe Asp Ala

675                 680                 685

Gly Ala Gly Thr Gly Gly Pro Ala Pro Gly Val Gly Ala Ala Ala Thr
    690                 695                 700

Ala Gly Ala Phe Ala Gly Ala Gly Pro Gly Gly Ala Asn Ala Gly Gly
705                 710                 715                 720

Asn Ala Ala Ala Gly Ala Gly Pro Gly Val Gly Pro Gly Gly Leu Gly
                725                 730                 735

Gly Leu Gly Gly Leu Gly Ala Gly Gly Leu Gly Gly Gly Leu Gly Gly
            740                 745                 750

Gly Leu Gly Gly Leu Gly Gly Ala Gly Gly Leu Gly Gly Gly Leu Gly
        755                 760                 765

Gly Leu Gly Gly Gly Leu Gly Gly Gly Leu Gly Gly Leu Gly Gly Gly
    770                 775                 780

Ala Gly Gly Ala Gly Ala Gly Gly Asn Gly Gly Ala Gly Ala Gly Gly
785                 790                 795                 800

Ala Gly Gly Asn Gly Gly Gly Ser Ala Ala Ala Arg Ala Ala Ala Gln
                805                 810                 815

Ala Ala Ala Ala Ala Gly Gly Asn Gly Gly Ala Ala Gln Ala Ala Ala
            820                 825                 830

Gln Ala Ala Ala Ser Ala Ala Ala Asn Ser Gly Leu Gly Ala Gly Ala
        835                 840                 845

Ala Arg Ala Ala Ala Ser Ala Ala Ala Arg Ala Thr Val Thr Gly His
    850                 855                 860

Gly Ser Gly Thr Ala Ala Ala Ala Ala Asn Ala Ala Ala Gln Ala His
865                 870                 875                 880

Ala Ala Thr Arg Gly Gln Gly Gly Ser His Ala His Ala Ala Ala Ala
                885                 890                 895

Ala His Ala Ala Ala Ser Ser Val Ile His Gly Gly Asp Tyr His Gly
            900                 905                 910

Asn Asp Ala Gly Tyr His Lys Pro Gly Tyr
        915                 920

<210>  9
<211>  19
<212>  PRT
<213>  Saccharomyces cerevisiae

<400>  9

Met Arg Phe Pro Ser Ile Phe Thr Ala Val Leu Phe Ala Ala Ser Ser
1               5                   10                  15

Ala Leu Ala

<210>  10
<211>  952
<212>  PRT
<213>  Mytilus edulis

<400>  10

Met Val Arg Phe Ser Leu Ala Ser Val Leu Leu Leu Ala Val Thr Ser
1               5                   10                  15

Thr Ala Phe Ala Gly Pro Val Ser Asp Tyr Gly Gly Gly Gly Ile Lys
            20                  25                  30

Val Val Pro Tyr His Gly Gly Gly Gly Gly Gly Gly Gly Gly Gly Gly
        35                  40                  45

Gly Gly His Gly Gly Ser Phe Arg Asn Gly Arg His Gly Gly Ile Gly
    50                  55                  60

Gly Ile Gly Gly Gly Ser Ser His Ala His Ala His Ser Ser Ala Ser
65                  70                  75                  80

Ala His Val His His Phe Gly Pro Gly Gly Ser Ser His Ala Ser Ala
                85                  90                  95

Gly Ser Ser Ser His Ala Ser Ala Ser His Asn Gly Leu Gly Gly Gly
            100                 105                 110

Ser Ala His Ala His Ser Ser Ser Ser Ala Asn Ala His Ser Gly Gly
        115                 120                 125

Phe Gly Gly Phe Gly Gly Ile Gly Gly Ile Gly Gly Ile Gly Pro Gly
    130                 135                 140

Gly Ser Val Gly Gly Gly Ile Gly Pro Gly Gly Ser Val Gly Gly Gly
145                 150                 155                 160

Ile Gly Gly Ile Gly Gly Ile Gly Gly Gly Gly Gly Pro Gly Gly Asn

                165                 170                 175

Gly Gly Ile Gly Phe Gly Pro Gly Phe Gly Gly Gly Phe Gly Pro Gly
            180                 185                 190

Ser Ser Ala Ser Gly Ser Gly Ser Gly Ser Ala Phe Gly Gly Pro Gly
        195                 200                 205

Gly Ser Ser Ala Ser Ala Asn Ala Ala Ala Arg Ala Asn Ala Asn Gly
    210                 215                 220

Gly Gly Gly Phe Gly Gly Pro Gly Thr Pro Gly Asn Ser Gly Pro Pro
225                 230                 235                 240

Gly Gln Pro Gly Leu Pro Gly Ala Pro Gly Gln Pro Gly Arg Pro Gly
                245                 250                 255

Ser Thr Pro Pro Gly Arg Leu Gly Asn Pro Gly Pro Pro Gly Gln Pro
            260                 265                 270

Gly Asn Pro Gly Arg Pro Gly Ser Ser Gly Arg Pro Gly Gly Ser Gly
        275                 280                 285

Gln Pro Gly Gly Pro Gly Arg Pro Gly Thr Pro Gly Lys Pro Gly Asn
    290                 295                 300

Arg Gly Gln Pro Gly Gln Pro Gly Gly Pro Gly Gln Pro Gly His Pro
305                 310                 315                 320

Gly Ala Gly Gly Gln Pro Gly Arg Asn Gly Asn Pro Gly Asn Pro Gly
                325                 330                 335

Lys Pro Gly Thr Pro Gly His Pro Gly Thr Ala Gly Ser Arg Gly Met
            340                 345                 350

Pro Gly Thr Pro Gly Thr Pro Gly Gln Pro Gly Ile Pro Gly Thr Val
        355                 360                 365

Gly Gly Arg Gly Pro Arg Gly Pro Ala Gly Ile Ile Gly Leu Ile Gly
    370                 375                 380

Pro Lys Gly Asn Pro Gly Glu Pro Gly Asn Pro Gly Ala Pro Gly Gly
385                 390                 395                 400

Pro Gly Ser Thr Gly Pro Gln Gly Pro Gln Gly Pro Ala Gly Gly Pro
                405                 410                 415

Gly Ala Ser Gly Gly Pro Gly Asp Lys Gly Ala Pro Gly Thr Pro Gly
            420                 425                 430

Gly Thr Gly Pro Arg Gly Pro Ile Gly Pro Ser Gly Pro Ser Gly Ala
        435                 440                 445

Pro Gly Asp Gln Gly Pro Gln Gly Gly Arg Gly Thr Pro Gly Leu Ala
    450                 455                 460

Gly Lys Pro Gly Pro Lys Gly Leu Gln Gly Ser Asn Gly Glu Val Gly
465                 470                 475                 480

Pro Gln Gly Pro Ser Gly Pro Ala Gly Pro Gln Gly Pro Gln Gly Lys
                485                 490                 495

Asn Gly Val Lys Gly Ala Ala Gly Asp Gln Gly Ala Arg Gly Pro Glu
            500                 505                 510

Gly Lys Ala Gly Pro Ala Gly Pro Gln Gly Glu Thr Gly Pro Lys Gly
        515                 520                 525

Pro Thr Gly Ala Gln Gly Pro Ala Gly Pro Ala Gly Pro Ser Gly Glu
    530                 535                 540

Gln Gly Pro Gly Gly Glu Arg Gly Gly Gln Gly Pro Gln Gly Ala Glu
545                 550                 555                 560

Gly Pro Ser Gly Pro Ala Gly Pro Arg Gly Pro Ala Gly Ser Gln Gly
                565                 570                 575

Pro Ser Gly Glu Arg Gly Glu Pro Gly Ala Pro Gly Lys Lys Gly Pro
            580                 585                 590

Asn Gly Asp Arg Gly Asn Gln Gly Ser Pro Gly Ala Pro Gly Lys Asn
        595                 600                 605

Gly Ala Arg Gly Asn Arg Gly Ser Arg Gly Ser Asn Gly Ser Pro Gly
    610                 615                 620

Arg Ser Gly Ser Pro Gly Ser Arg Gly Lys Pro Gly Pro Gln Gly Pro
625                 630                 635                 640

His Gly Pro Arg Gly Leu Arg Gly Ser Pro Gly Gln Lys Gly Pro Arg
                645                 650                 655

Gly Asp Gln Gly Ala Pro Gly Val Ile Arg Ile Val Ile Asp Asp Gln
            660                 665                 670

Arg Thr Gly Pro Glu Val Ala Glu Phe Pro Gly Phe Gly Gly Phe Gly
        675                 680                 685

Gly Ala Ser Ala Asn Ala Ala Ser Ser Ala Asn Ala Phe Ala Gly Gly
    690                 695                 700

Pro Gly Gly Ser Ala Gly Ala Gly Ser Ser Ser Gly Ala Asn Ala Asn
705                 710                 715                 720

Ala Gly Gly Phe Pro Phe Gly Gly Ala Gly Gly Gly Pro Gly Ala Ala
                725                 730                 735

Gly Gly Pro Gly Gly Ala Gly Gly Pro Gly Gly Val Gly Gly Gly Val
            740                 745                 750

Gly Gly Gly Pro Gly Gly Val Gly Gly Gly Val Gly Gly Gly Pro Gly
        755                 760                 765

Gly Val Gly Gly Gly Pro Gly Gly Ala Gly Pro Gly Gly Ala Gly Gly
    770                 775                 780

Phe Gly Pro Gly Gly Ala Gly Gly Phe Gly Gly Phe Gly Gly Gly Ser
785                 790                 795                 800

Ser Ala Gly Ala Ser Ser Ser Gly Ser Ala Ser Ala Ser Asn Gly Gly
                805                 810                 815

Pro Phe Gly Val Leu Asn Val Gly Pro Gly Gly Arg Ile Gly Gly Gly
            820                 825                 830

Ser Ala Ser Ala Ser Ala Ala Ser Arg Ala His Ala His Phe Gly Gly
        835                 840                 845

Gly Ser Ser Ala Gly Ala Ser Ser Ser Gly Ser Ala Ser Ala Ser Asn
    850                 855                 860

Gly Gly Pro Phe Gly Val Leu Asn Val Gly Pro Gly Gly Arg Ile Gly
865                 870                 875                 880

Gly Gly Ser Ala Ser Ala Ser Ala Ala Ser Arg Ala His Ala His Ala
                885                 890                 895

Phe Gly Gly Leu Gly Gly Gly Ser Ala Ser Ala Gly Ser His Ser Ser
            900                 905                 910

Ser Ser Ser His Ser Phe Gly Gly His Val Phe His Ser Val Thr His
        915                 920                 925

His Gly Gly Pro Ser His Val Ser Ser Gly Gly His Gly Gly His Gly
    930                 935                 940

Gly Gly Pro Tyr Lys Pro Gly Tyr
945                 950

## Description

The present invention is directed to a recombinant mussel byssus protein, an isolated nucleic acid encoding same and a host cell, which has been transformed with an expression vector containing said nucleic acid. The present invention is further directed to a kit of parts or a co-expression system for a mussel byssus protein and to a method of producing said recombinant mussel byssus protein. Furthermore, the invention pertains to a method of producing threads from said proteins as well as threads obtainable therefrom and their use in various fields of technology and medicine.

Marine mussels are found in the turbulent habitat of the inter-tidal zone and here, marine mussels have been very successful in colonizing rocks, which are exposed to wind and waves. This success is partially due to a unique anchorage by which they fix themselves on the solid surfaces of the rocks. A part of this anchorage is a fibrilar structure, known as "byssus" or also known as "mussel silk". The byssus provides mussels with the necessary tenacity to survive the incessant buffeting of waves by attaching to rocks or hard surfaces.

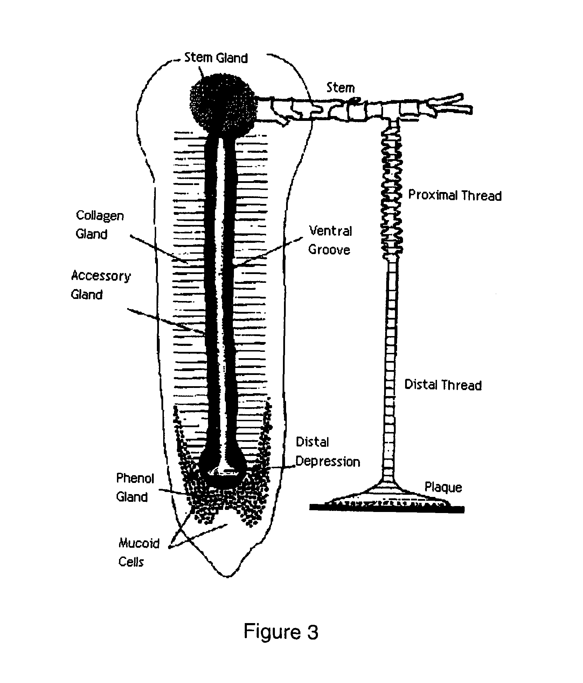

The mussel byssus is completely consisting of extra-cellular matrix which is forming a bundle of short threads that resemble tiny tendons [2]. Byssus threads show unusual mechanical properties, since they resemble soft rubber at one end and rigid nylon at the other and these properties are found with a seamless and gradual transition [4]. Byssal threads are also elastomeric: they are able to withstand significant deformations without rupture and can return to their original state, when the stress is removed [5]. At the distal end, the byssus threads are fixed by adhesive plaques at the rock. At the proximal ends, the byssus threads are combined to a so-called byssus stem, which is anchored at the base of the mussel foot (see Figure 3).

The byssus threads of marine mussels are elastomeric fibers with a great capacity of absorbing and dissipating energy. Up to 70% of the total absorbed energy can be dissipated in the byssus. In *Mytilus* species (*M. edulis* and *M. galoprovincialis*), each new thread has dimensions of a few centimeters in length and less than 0.1 cm in diameter and is produced in ca. 5 minutes in the ventral groove of the foot by a process akin to reaction injection molding [3].

Morphologically, the byssus is divided into four sections (from proximal to distal): root, stem, thread and plaque or pad. Furthermore, the thread is further subdivided into proximal and distal portions according to appearance, i.e. smooth and stiff for the distal, soft and weaker for the proximal portion.

Byssus threads are elastomeric. The Young's modulus is low (in the range of from 10-500 MPa), the extensibility can be as high as 200% and there is restorative recall. In common with other protein elastomers as elastin, resiline and abductine, byssus threads are quite tough. Thoughness and energy dissipation are both crucial properties for holdfasts. Energy dissipation in fibers subjected to cyclic stress-strain-analysis is frequently normalized with respect to the total absorbed energy and reported as hysteresis or percentage hysteresis.

The stress-strain cycle for one thread has been dissected into separate mechanical contributions for the distal and proximal portions of the thread. As mentioned above, of these, the distal portion is stronger, stiffer and superior at damping whereas the proximal portion is softer and weaker with a lower, but still significant hysteresis.

The mechanical properties of byssus threads are further complicated by time- and strain-dependent behavior. It was demonstrated that, when strained beyond its yield point, the distal portion exhibited a schematic stress softening, i.e. the initial modulus of the second cycle was reduced to about 20% of the modulus in the first cycle (500-80 MPa). The complete recovery of the modulus of the first cycle was slow, e.g. longer than 24h but significant partial recovery can occur within 1h (30% of the original values). The proximal portion also shows a tendency to change stiffness with cyclic loading. In this case, there is strain-stiffening from an initial modulus of 35 MPa to an asymptotic leveling at 50 MPa, an increase of about 40%.

MASCOLO and WAITE (1986) first identified chemical gradients in byssus threads in *Mytilus.* After treatment of the threads with pepsin, two pepsin-resistant collagen fragments, called ColP and ColD, having molecular weights of 50 kDa and 60 kDa, respectively were identified. ColP can be found predominantly in the proximal area and is hardly to be found in the distal area. In contrast, the amount of ColD increases in the distal part to approximately 100% (LUCAS et al., 2002; QIN & WAITE, 1995). In the byssus thread as well as in the mussel foot, there is a further collagen-like protein which takes part in the construction of the thread structure. This additional protein is called ColNG (NG = no gradient), and is, in contrast to ColD and ColP, evenly distributed throughout the whole thread. Its physiological function presumably is being an adapter between the two other thread collagens (QIN & WAITE, 1998).

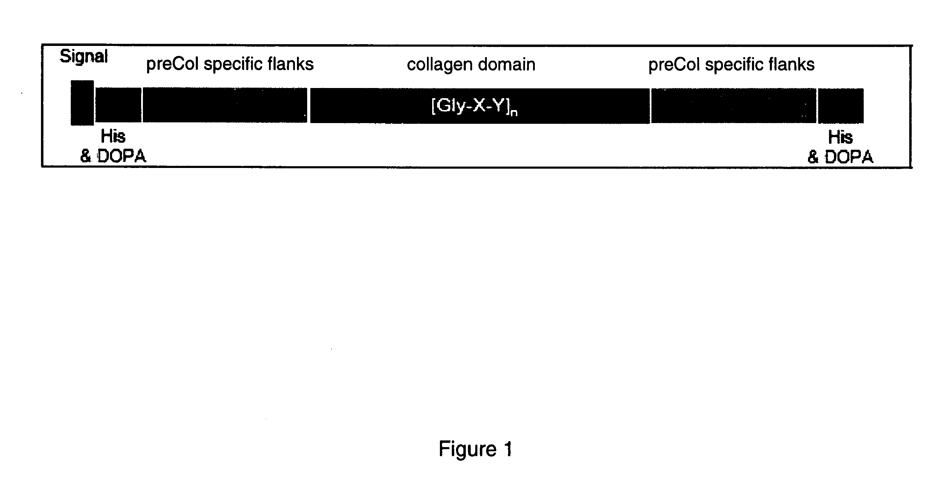

The Pepsin-cleaved fragments ColD and P originate from the so-called preCollagens P and D. Both preCol's (i.e. D and P) from *M. edulis* are characterized by a common basic structure: a central collagen helix which is flanked by different flanking regions, which are each terminated by a histidine and DOPA rich terminus (see Figure 1).

The mechanism for the assembly of byssus collagens into fibers has been an elusive aspect of the byssus biochemistry. It is well recognized that the collagens undergo stabilization via cross-linking; however the chemistry is still not well understood. There are two distinct cross-linking possibilities: metal complexation and covalent bond formation between collagen units [8, 9]. Metal complexation is suggested by the high levels of iron, copper, nickel and zinc found in byssus and by the occurrence of metal-binding histidine-rich sequences in both terminals of the byssal proteins. Moreover, DOPA is present in both the termini of all PreCol's. Peptidyl-DOPA provides excellent metal binding sites and peptidyl-DOPA-Fe(III) chelates have been reported in the marine adhesive plaque mefp-1 [10]. Further, it has been shown that removal of metal ions from byssal fiber by EDTA reduces the yield strength of the fiber. Covalent cross-links have also been observed. They are generally formed by oxidative coupling between tyrosines, DOPA and cysteines. In a study of byssus stressed by conditions of high flow and aeration, the primary product of oxidation was found to be 5,5'-diDOPA[11]. Other possible coupling products like the Michael-type addition of lysines to oxidized DOPA have not been found [7].

Like "normal" collagen, each mussel collagen has a signal sequence of 20 amino acids which make sure that the alpha-chains are transported into the endoplasmatic reticulum. There, three identical alpha-chains assemble to a homotrimer. The ColD alpha-chain, which means the pepsin-cleaved preColD, has a molecular mass of 60kDa by SDS-PAGE and 47 kDa by MALDI-TOF mass spectometry (QIN et al., 1997). The alpha-chain of ColP, which means the pepsin-cleaved preColP, has a molecular mass of 55 kDa (by SDS-PAGE) and 40 kDa (MALDI-TOF), respectively (COYNE et al., 1997). The precursors of the alpha-chain are named preColD and preColP and have molecular masses of 95 and 97 kDa by means of SDS-PAGE analysis and 75 and 80 kDa respectively by analysis with MALDI-TOF mass spectometry (COYNE et al., 1997; QIN et al., 1997). Both collagens have characteristics which are typical for collagen type I-III. Both have an amount of more than 34 % of glycine and show a proline and hydroxyproline content of combined 20% within the collagen domain.

The flanking regions fully correspond to other structural proteins, namely elastin (preColP) and silk-fibroin (preColD). This structural construction gives an explanation for the mechanical behavior of mussel byssi. For this reason, it would be highly relevant to recombinantly produce the underlying mussel byssus collagens in order to use these extraordinary natural materials as building blocks in new technological applications.

The development of materials having defined characteristics, in particular of materials which are capable of regenerate themselves following stress or overloading has been of high interest in the material sciences for a long time. Composite structures are of gaining interest in technology, in particular for electronic components and devices, energy converters and other materials. By combination of materials having different mechanical characteristics, structural interfaces will be formed causing new technological problems.

Thus, for many applications it would highly desirable to provide a graduated structure thereby reducing the overall load of the material.

Furthermore, the use and application of mussel collagens in medicine is of great interest because of the high potential biocompatibility. Based on this, medical transplants and tissues could be generated having a high degree of immunocompatibility. The production of recombinant mussel collagens is an interesting and important technical problem which has to be solved before technical applications of mussel collagens may be envisioned.

Therefore it is an object underlying the present invention to provide recombinant mussel byssus proteins having enhanced characteristics as, in particular, improved capability of being expressed in high yield and good strength and flexibility. It is a further object of the present invention to provide recombinant mussel byssus proteins which can be specifically adapted to the required application by specific arrangement of the building blocks on which they are based to provide a graduated structure. Furthermore, it is an object of the present invention to provide expression vectors coding for recombinant mussel byssus proteins, which can be conveniently expressed in already known eucaryotic expression systems. Additionally, it is an object of the present invention to provide improved paper, textile and leather products. Additional objects are to provide new proteins and further materials based on recombinant mussel byssus proteins such as spheres, nanofibrils, hydrogels, threads, foams, films for use in biotechnology, medicine, pharmaceutical and food applications, cosmetics, in electronic devices and for other commercial purposes.

These objects are solved by the subject-matter of the independent claims. Preferred embodiments are set forth in the dependent claims.

Up to now, the expression of recombinant mussel byssus proteins has never been shown. This might be at least partially due to the complex process of expressing those proteins and threads made therefrom.

The present invention in particular is directed to the following aspects and embodiments:

According to a first aspect, a recombinant mussel byssus protein is provided comprising or consisting of one or more fragments of a collagen domain flanked by elastin or silk fibroin.

The recombinant mussel byssus protein of the invention is composed of one or more types of building blocks, which provide different characteristics to the protein formed: as mentioned above, elastin and silk-fibroin have certain mechanical characteristics, which can give an explanation for the mechanical behavior of mussel byssi and, thus, also for the design of recombinantly produced mussel byssus proteins.

Therefore, the fragments of the present invention can be used as one single type of fragment only, or, as an alternative, the recombinant protein can comprise two or more different fragments. For example, if great elasticity is wanted, the protein may only or predominantly comprise fragments of collagen flanked by elastin. If great stiffness and strength is required, the protein may comprise fragments of collagen flanked by silk-fibroin. As a further and preferred alternative, the protein may comprise a mixture of both types of fragments, for example forming a gradient from one region to the other. Thus, a protein/thread can be formed having specifically adapted configurations, i.e. parts having higher elasticity and parts having higher stiffness etc.

The term "flanked" means that elastin (or silk-fibroin) is present on both sides of the collagen domain.

The above fragments may be naturally derived, for example, the fragments may be obtained from *Mytilus sp.,* preferably from *M. edulis, M. galloprovincialis, M. californians,* or *Geukeria demissa.*

According to a preferred embodiment, the recombinant mussel byssus protein of the invention comprises or consists of one or more of the fragments preColP and/or preColD or variants thereof. These fragments have been outlined above. Both preCol's (i.e. D and P) are derived from *M. edulis* and are characterized by a common basic structure: a central collagen helix which is flanked by different flanking regions, which are each terminated by a histidine and DOPA rich terminus (see Figure 1). The flanking regions fully correspond to known structural proteins, namely elastin (preColP) and silk-fibroin (preColD).

The sequences of preColP and preColD are translated from the respective nucleic acids. Therefore, whenever amino acids are recited herein in the following, they are referring to preColP and preColD and these sequences will be used in the various technological applications mentioned hereinabove. The nucleic acid sequences mentioned herein in the first place are directed to preColP and preColD encoding sequences.

According to a further embodiment, the recombinant protein of the invention comprises or consists of one or more fragments of SEQ ID NO: 3 and/or 4 or variants thereof. The Seq ID No's reflect the sequences of preColP and preColD.

As mentioned above, the present invention also comprises variants of those amino acid sequences. For example, said variants may contain one or more substitutions, insertions and/or deletions when compared to the amino acid sequences mentioned above.

In particular variants of the protein, for example deletions, insertions and/or substitutions in the sequence, which cause so-called "silent" changes, are considered to be part of the invention.

Preferably are such amino acid substitutions the result of substitutions which substitute one amino acid with a similar amino acid with similar structural and/or chemical properties, i.e. conservative amino acid substitutions.

Amino acid substitutions can be performed on the basis of similarity in polarity, charges, solubility, hydrophobic, hydrophilic, and/or amphipathic (amphiphil) nature of the involved residues. Examples for hydrophobic amino acids are alanine, leucine, isoleucine, valine, proline, phenylalanine, tryptophan and methionine. Polar, neutral amino acids include glycine, serine, threonine, cysteine, thyrosine, asparagine and glutamine. Positively (basic) charged amino acids include arginine, lysine and histidine. And negatively charged amino acids include aspartic acid and glutamic acid.

"Insertions" or "deletions" usually range from one to five amino acids. The allowed degree of variation can be experimentally determined via methodically applied insertions, deletions or substitutions of amino acids in a polypeptide molecule using recombinant DNA methods. The resulting variants can be tested for their characteristics, in particular their mechanical characteristics.

It is noted that the term "variant" as used herein also comprises the above amino acid sequences of preColP and preColD, wherein the first 19 amino acids constituting the original mussel signal sequence were replaced by other signal sequences. A preferred example hereof is replacement of the mussel signal sequence by signal sequence alpha MF (SEQ ID NO: 10: "MRFPSIFTAV LFAASSALA"). This signal sequence in particular is suitable for expression of the nucleic acids in yeasts.

In a second aspect, the present invention provides an isolated nucleic acid encoding the recombinant protein as defined above. The term "isolated" as used herein with reference to nucleic acids refers to a naturally-occurring nucleic acid that is not immediately contiguous with both of the sequences with which it is immediately contiguous (one on the 5'end and one on the 3'end) in the naturally-occurring genome of the organism from which it is derived.

For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" also includes any non-naturally-occurring nucleic acid since non-naturally-occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acids can be independent of other sequences, or incorporated into a vector, an autonomously replicating plasmid, a virus (e. g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

It will be apparent to those of skill in the art that a nucleic acid existing among hundreds to millions of other nucleic acid molecules within, for example, cDNA or genomic libraries, or gel slices containing a genomic DNA restriction digest is not to be considered an isolated nucleic acid.

A nucleic acid encoding the above amino acids may be a nucleic acid sequence coding for the mature or the immature amino acid sequence of the recombinant mussel byssus protein.

In a preferred embodiment, the isolated nucleic acid comprises or consists of the nucleic acid of SEQ ID NO: 1 and/or 2 or variants thereof. These variants are each defined as having one or more substitutions, insertions and/or deletions as compared to the sequences of SEQ ID NO: 1 or 2, provided that said variants hybridize under moderately stringent or stringent conditions to a nucleic acid which comprises the sequence of SEQ ID NO: 1 or 2, or provided that said variants comprise nucleic acid changes due to the degeneracy of the genetic code, which code for the same or a functionally equivalent amino acid as the nucleic acid sequence of SEQ ID NO: 1 or 2.

As mentioned above, the present invention also encompasses a variant of said nucleic acids, wherein the nucleic acids coding for the first 19 amino acids (signal sequence) were replaced, preferably by the yeast signal sequence MFa (SEQ ID NO: 10).

Stringency of hybridization, as used herein, refers to conditions under which polynucleotide duplexes are stable. As known to those of skill in the art, the stability of duplex is a function of sodium ion concentration and temperature (see, for example, Sambrook et al., Molecular Cloning: A Laboratory Manual 2nd Ed. (Cold Spring Harbor Laboratory, (1989)). Stringency levels used to hybridize can be readily varied by those of skill in the art.

Stringent washing conditions mean 0.2 x SSC (0.03 M NaCl, 0.003 M sodium citrate, pH 7)/0.1% SDS at 65°C. For shorter fragments, e.g. oligonucleotides up to 30 nucleotides, the hybridization temperature is below 65°C, for example at 50°C, preferably above 55°C, but below 65°C. Stringent hybridization temperatures are dependent on the size or length, respectively of the nucleic acid and their nucleic acid composition and will be experimentally determined by the skilled artisan. Moderate stringent hybridization temperatures are for example 42°C und washing conditions with 0.2 x SSC/0.1% SDS at 42°C.

In a further aspect, the present invention provides an expression vector comprising the nucleic acid sequence as defined above and one or more regulatory elements. The expression vector must be suitable for expression in eucaryotic cells.

Preferably, the regulatory elements contain a promoter selected from constitutive or inducible promoters, more specifically from GPD, GAL4, CUP1, MET25, GAL1 or GAL1-10.

In a further embodiment, the expression vector is a plasmid.

The invention is further directed to an expression vector comprising a nucleic acid coding for prolyl-4-hydroxylase, wherein the nucleic acid is linked to a signal sequence for efficient transport of said sequence to the ER.

Due to the complexity of the biosynthesis of collagen, for the recombinant synthesis of collagen-like proteins, some factors have to be considered, the most important one being the posttranslational modification in the endoplasmatic reticulum (ER) of proline to hydroxyproline by prolyl-4 hydroxylase, a tetrameric enzyme, which is composed of the two sub-units of alpha-PH (= P4HA) and PDI (= P4HB) (BULLEID et al., 2000). For this reason, procaryotic expression systems, for example bacterial expression systems, may not be used in the present invention.

Eucaryotic systems, however, seem to be appropriate for this purpose. As an example, yeasts on the one hand offer the cell compartmentation which is required for the synthesis of collagen, on the other hand, however, they are lacking the enzyme prolyl-hydroxylase (P4H) which is required for the synthesis of collagen. However, it could be shown for *Saccharomyces cerevisae* and *Pichia pastoris,* which do not possess P4H, that human P4H subunits can be produced recombinantly and can be correctly folded. Apart therefrom, it could be shown for these yeast strains that by co-expression of both human P4H subunits, the synthesis of human collagen type I-III is possible and folded, stabile collagen triple helizes are formed.

Interestingly, the co-expression of the genes of both P4H subunits is sufficient for the formation of a stable triple helix in yeast and no further enzymes or folding promoters or chaperones specific for collagen are required, as for example Hsp47, or in other words, the chaperones which are inherent to yeast are sufficiently "active". Human collagens, recombinantly produced in yeast possessed the same content of hydroxyproline and, furthermore, are identical in respect to many other characteristics compared to native collagens.

By efficient transport in the ER of yeast, signal sequences of the co-expressed P4H subunits play an important role. A maximum sufficiency of localization can be achieved by replacing the human with a yeast signal sequence, for example from the *S. cerevisae* pheromone *mating factor alpha1* (MFa). The P4H subunits modified by the MFa signal sequence were effectively transported into the lumen of the ER.

Thus, in the expression vector of the invention, the signal sequence is a host, preferably yeast, specific signal sequence, preferably *mating factor alpha 1* (MFa) of *S. cerevisiae.*

In a fourth aspect, a kit of parts or a co-expression system comprising the following constituents is provided:
a) the expression vector encoding the recombinant mussel byssus protein as defined hereinabove; and
b) an expression vector coding for prolyl-4-hydroxylase, wherein the nucleic acid is linked to a signal sequence for efficient transport of said sequence to the ER.

This kit of parts or co-expression system may be efficiently used in expressing the recombinant mussel byssus protein in the host cells mentioned below, in particular in yeast cells.

In a fifth aspect, the invention discloses a host cell, which has been transformed with the expression vectors as defined above or with the above co-expression system (kit-of-parts).

As already mentioned, this host cell preferably is a eukaryotic cell, more preferably a mammalian cell, plant cell, yeast cell or an insect cell.

As a yeast cell, *S. cerevisiae, Schizosaccharomyces pombe, Pichia pastoris, Candida albicans,* or *Hansenula polymorpha* cells might be used.

Suitable mammalian cells are selected from CHO, COS, HeLa, 293T, HEH or BHK cells. As an insect cell Lepidoptera insect cells, preferably from Spodoptera frugiperda and from Trichoplusia ni may be employed, preferably Sf9, Sf21 or high five cells.

Also plant cells are envisioned in the present invention which are derived from tobacco, potato, corn, pea and tomato.

In a still further aspect, a method of producing recombinant mussel byssus proteins is disclosed comprising the steps of:
a) providing a host cell as defined hereinabove;
b) transforming said host cell with an expression vector or the co-expression system explained above;
c) expressing recombinant mussel byssus protein from said host cell under suitable conditions; and
d) recovering said mussel byssus protein.

Furthermore, a method for producing threads from recombinant mussel byssus protein is provided, comprising the following steps:
a) providing recombinant mussel byssus protein produced in accordance with the above method, and
b) spinning or moulding said protein into threads by a suitable method.

The spinning may preferably be done by electrospinning. Electrospinning is a fiber formation technique that uses electrostatic forces to create continuous, nanometer diameter fibers. A wide variety of natural and artificial polymers have been electrospun from the solution and melt phase and are of interest for an assortment of application areas that require high surface area materials (filtration membranes and biomedical devices).

An additional aspect of the invention is a thread obtainable by the above method.

The proteins/threads of the invention find application preferably in the field of biotechnology and/or medicine.

For example, they might be used for the manufacture of wound closure or coverage systems or suture materials. Furthermore, the proteins/threads may preferably be used for the manufacture of replacement materials, preferably artificial cartilage or tendon materials.

Additionally, the threads/proteins of the invention can be used in the manufacture of medical devices such as medical adhesive strips, skin grafts, replacement ligaments, and surgical mesh; and in a wide range of industrial and commercial products, such as clothing fabric, bullet-proof vest lining, container fabric, bag or purse straps, cable, rope, adhesive binding material, non-adhesive binding material, strapping material, automotive covers and parts, aircraft construction material, weatherproofing material, flexible partition material, sports equipment; and, in fact, in nearly any use of fiber or fabric for which high tensile strength and elasticity are desired characteristics. Adaptability and use of the stable fiber product in other forms, such as a dry spray coating, bead-like particles, or use in a mixture with other compositions is also contemplated by the present invention.

It is explicitely noted that preferred applications of the mussel byssus collagens of the present invention are in the manufacture and processing of clothing fabric (textiles) and leather, automotive covers and parts, aircraft construction materials as well as in the manufacture and processing of paper.

The recombinant mussel byssus proteins of the present invention may be added to cellulose and keratin and collagen products and thus, the present invention is also directed to a paper or a skin care and hair care product, comprising cellulose and/or keratin and/or collagen and the proteins of the present invention. Papers and skin care and hair care products, in which the proteins of the present invention are incorporated are showing improved characteristics, in particular improved tensile strength or tear strength.

Furthermore, the recombinant mussel byssus proteins of the invention may be used as a coating for textile and leather products, thereby conferring stability and durability to the coated product. The proteins in particular show applicability for coating leather products, since in this case, tanning and its negative effects for environment can be avoided or at least reduced.

The invention is also directed to products containing said mussel byssus proteins, for example, wound closure or coverage systems, suture materials, replacement materials, preferably artificial cartilage or tendon materials, cosmetics, drug delivery vehicles, fabrics, textile, paper product, leather product, automotive parts or aircraft parts. In general, it is also directed to materials based on recombinant mussel byssus proteins such as spheres, nanofibrils, hydrogels, foams, films.

Unless otherwise defined, all technical and scientific terms used herein have the same meaning as commonly understood by one of ordinary skill in the art to which this invention pertains. All publications, patent applications, patents, and other references mentioned herein are incorporated by reference in their entirety. In case of conflict, the present specification, including definitions, will control. In addition, the examples are illustrative only and not intended to be limiting.

The invention is now further illustrated by examples and the accompanying drawings, which are showing the following:
- Fig. 1: is illustrating the general structure of mussel byssus collagens;

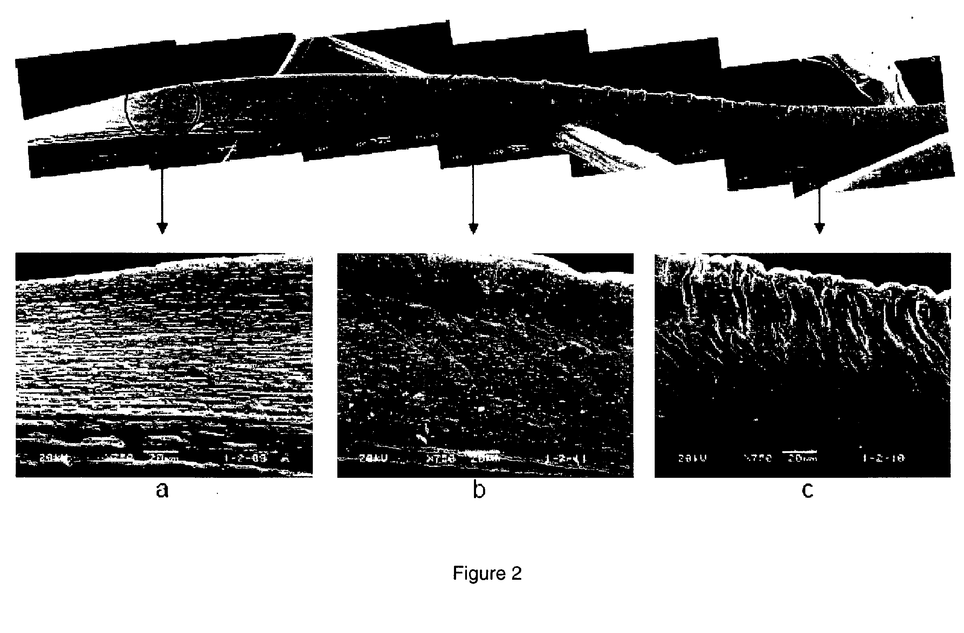

- Fig. 2: depicts a series of SEM images of byssus threads in direction distal to proximal - the marked portions are each enlarged below. a) distal; b) median; c) proximal;
- Fig. 3: shows the structure of mussel byssus;

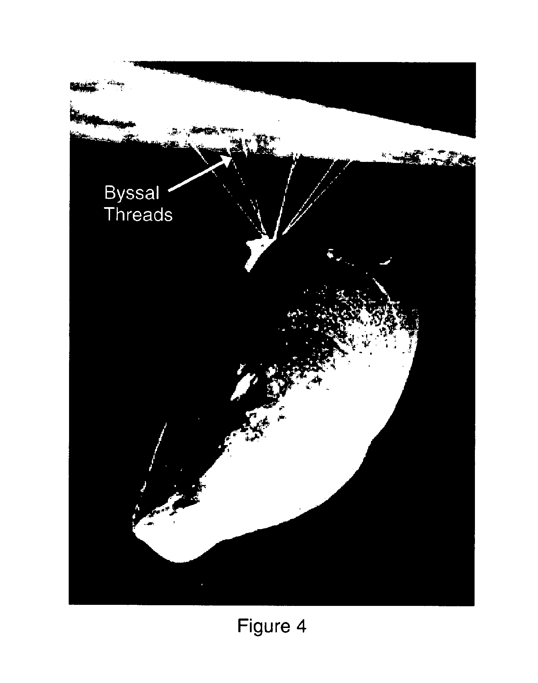

- Fig. 4: illustrates a mussel adhered to a solid surface by byssus threads;
- Fig. 5:: (A) Distribution of preCols in the thread. (B) Schematic of a collagenous subunit with flanking domains. Terminal regions denoted by diamonds are His-rich. DOPA is designated by Y. (C) Model of cross-linking interactions between axial and lateral preCols;
- Fig. 6:: Design of the P4H construct;
- Fig. 7:: Design of oligonucleotides to generate α-MF signal sequences ready to be cloned into respective expression plasmids;
- Fig. 8:: Cloning strategy for α-PH;

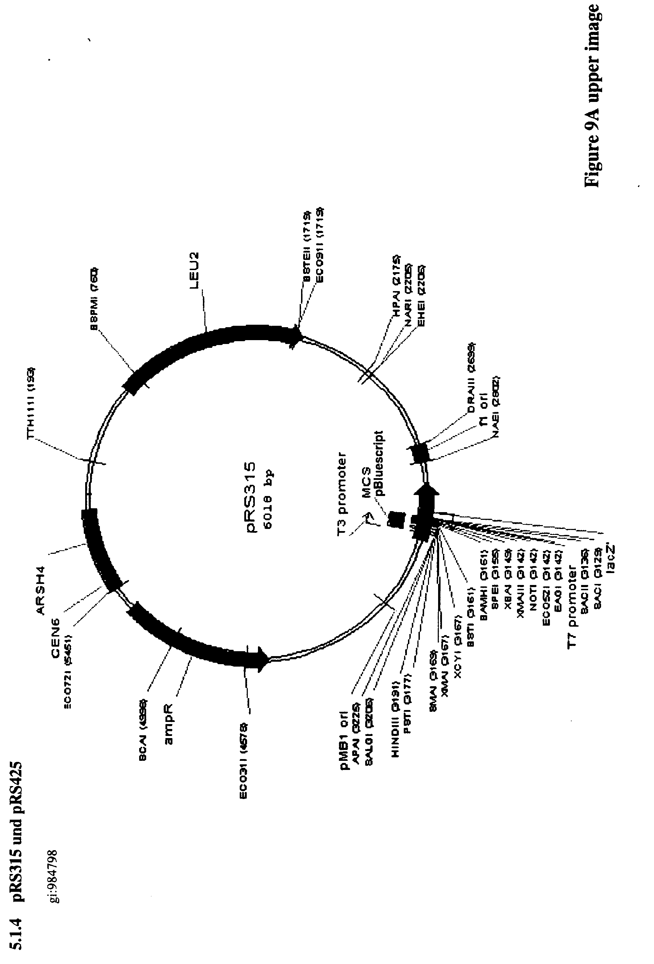

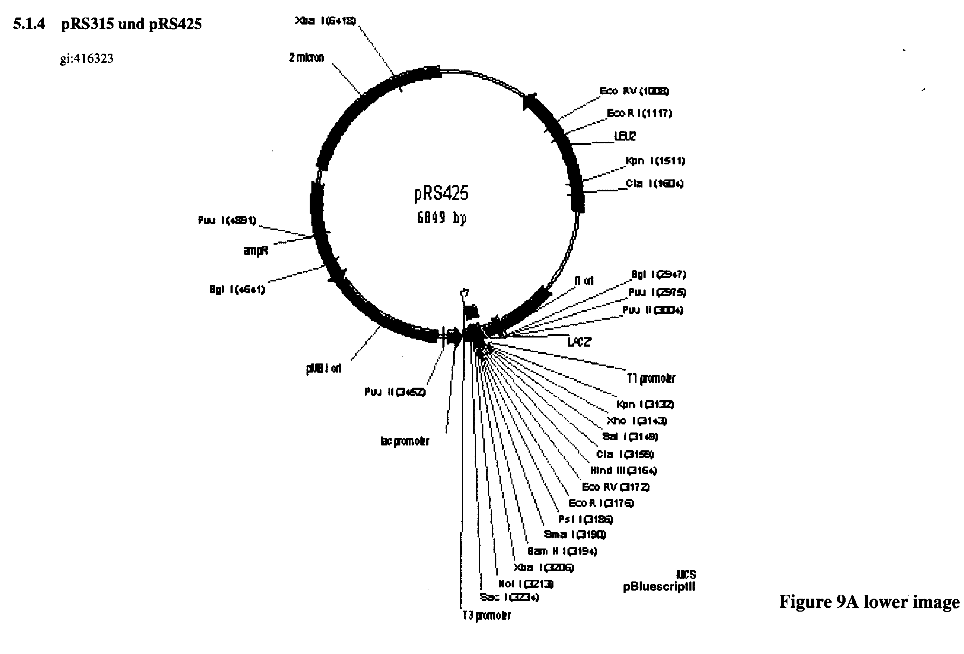

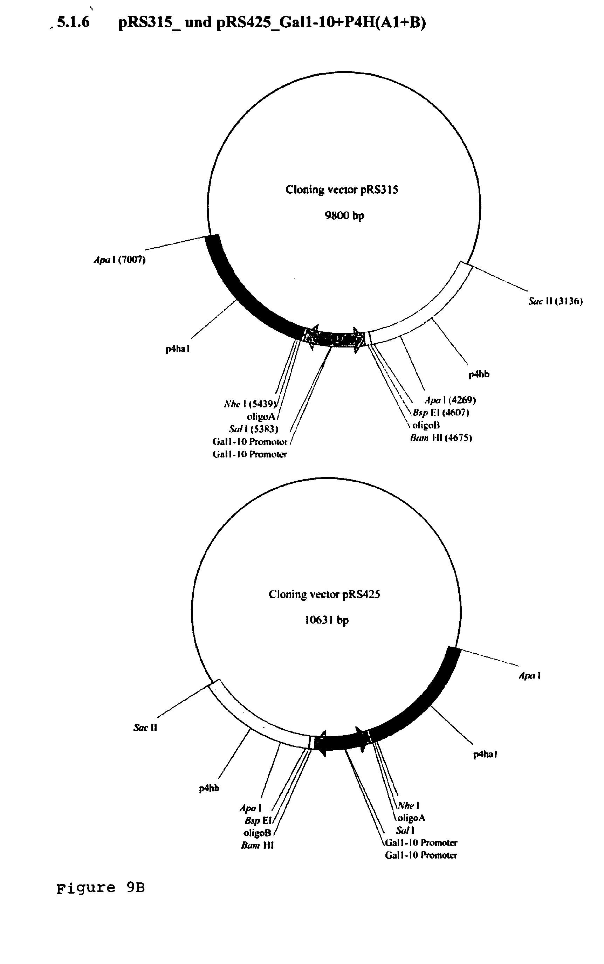

- Fig. 9:: Vector maps.

### Examples:

### Expression of Collagen proteins of Mussel Byssus in Yeast

Collagen synthesis in general reflects a complex biochemical process. The process requires e.g. post-translational modification of certain prolines of the respective collagens to 4-hydroxyproline in the ER by the enzyme Prolyl 4-hydroxylase (P4H). P4H, an α₂ß₂ tetramer in vertebrates, plays a central role in the synthesis of collagens. 4-hydroxyproline residues, generated by P4H, are essential for the folding of the newly synthesized collagen polypeptide chains into triple-helical collagen molecules [13]. It has been shown that co-expression of various types of human collagen with the two subunits of human P4H (α-PH and PDI) can be used for efficient expression of human collagen in yeast *Pichia pastoris* [12] and also in *S. cerevisiae* without the need of other collagen assembly co-factors or collagen-specific chaperones such as Hsp47 [14]. A similar strategy will be used in the present example for the expression of mussel collagen in *S. cerevisiae.*

### Human Prolyl-4-Hydroxylase expression construct

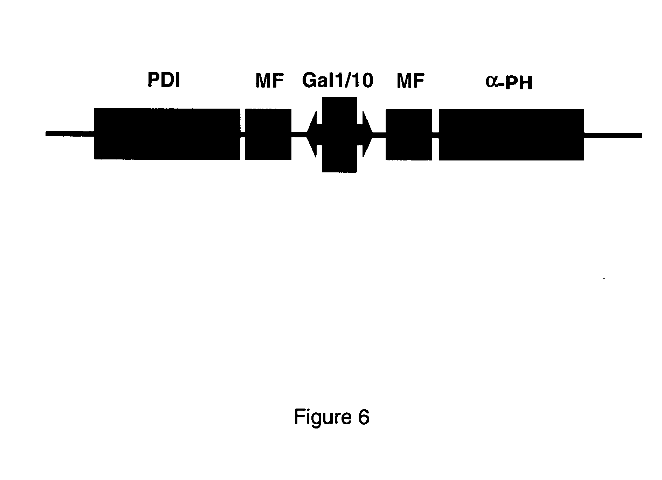

The construct of P4H requires the cloning of a signal sequence into the yeast vector adjacent to the genes for the two subunits of P4H, α-PH and PDI. Both genes are placed under the control of a bi-directional promotor, which is induced in the presence of Galactose (Gal 1/10) (see Figure 6). The signal sequence is required for translocation of P4H subunits into the ER, where they can assemble into the native tetramer. Maximum efficiency for localization has been achieved when the human signal sequence is replaced by yeast's own signal sequence of the mating factor α-MF [12]. See Figure 6 in this context.

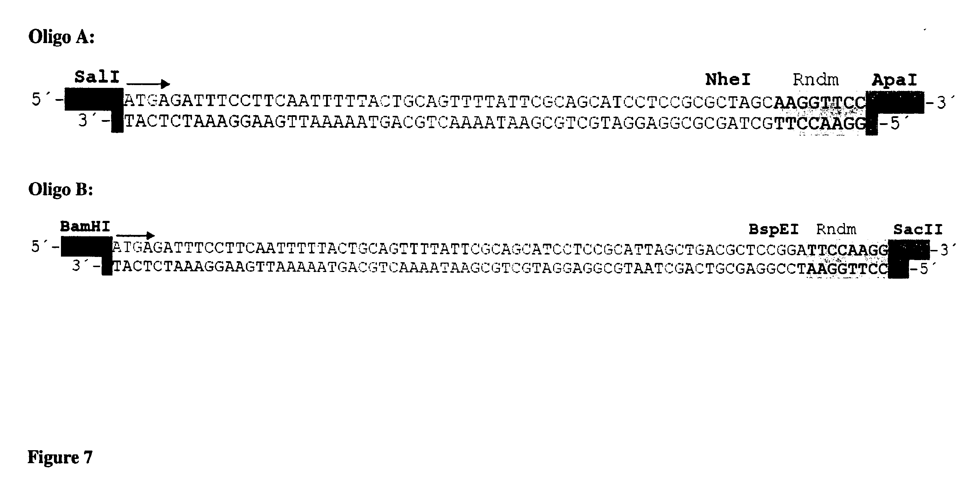

The gene for α-PH (without signal sequence) is amplified by PCR from a c-DNA library from HepG2 liver cells (provided by Professor Adamski, GSF Munich, Germany), while the c-DNA of the beta-subunit (PDI) (without signal sequence) will be amplified from an *E. coli* cloning vector (provided by Professor Neil Bulleid, University of Manchester, UK). For each gene a respective αMF signal sequence will be engineered based on two single stranded oligonucleotides. The oligos A and B are planned in a way (see figure 7) that after annealing the double stranded DNA can be directly cloned into respective vectors.

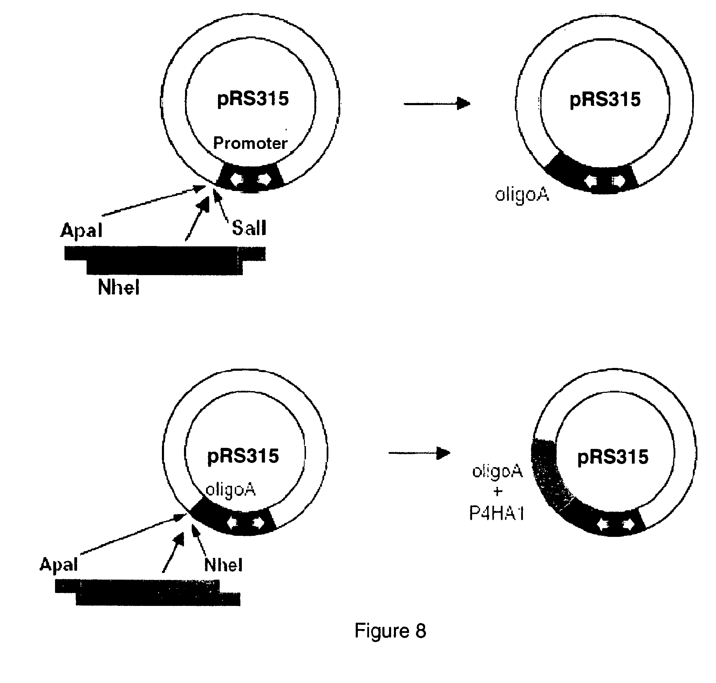

The cloning strategy for α-PH is shown as an example (Figure 8). Cloning of the cDNA of PDI will be performed in an identical way. Two different yeast vectors will be used: pRS315 (CEN, reflecting a single copy number plasmid) und pRS425 (2µ, reflecting a multi copy plasmid), both containing the bi-directional Gall/10 promotor, allowing the simultaneous expression of both subunits from one plasmid.

### Recombinant synthesis of preColD and preColP

Recombinant synthesis of preColD and preColP, two major protein components of Mussel Byssus is an example of the present invention. The c-DNA of PreColP and PreColD in *E. coli* cloning vectors has been obtained from Prof. Waite (UCSB, USA). The cDNA is amplified by PCR and cloned into different yeast expression vectors. The vectors differ in copy number per cell, as well as in the choice of the activator (either the constitutive promotor GPD or the inducible promotor GAL4). Also the original signal sequence will be replaced by the signal sequence of the yeast α-MF for maximum localization efficiency.

### Detection of ColP and ColD during recombinant synthesis

The test for the efficient recombinant synthesis of mussel collagen requires availability of polyclonal antibodies against mussel collagen. Preliminary tests with polyclonal antibodies against human collagen type I-III showed a very weak cross-reactivity against chemically denatured collagen from mussel byssus. This cross-reactivity is not sufficient to detect the levels of collagen present during the recombinant synthesis. Hence antibodies need to be raised against mussel collagen. In order to be able to raise antibodies, purified native mussel collagen is required. Byssus will be extracted from fresh mussels and purified using several chromatographic methods (reverse phase chromatography among others).

The purified protein samples, which contain both preColD and preColP, are used to immunize rabbits and generate antibodies.

### Biophysical studies on recombinant Collagen

Various physical methods can be used to characterize the individual proteins preColP/preColD and to evaluate the efficiency of fiber formation on self-assembly. These methods include far- and near-UV circular dichroism (CD), static and dynamic light scattering, fourier transformed infrared spectroscopy (FTIR), electron microscopy (EM), atomic force microscopy (AFM) and field flow fractionation (FFF).

### Characterization of individual preColP andpre ColD

CD and FTIR will be used to determine the secondary and tertiary structures of preColP and preColD. Their chemical and thermal stability will also be tested under various conditions. Data on the shape of the proteins involved in the collagen formation are provided by light scattering, by AFM and TEM.

### Evaluation of rate and efficiency of fiber formation

Secondary and tertiary structure of mussel byssus collagen are analyzed by CD and FTIR. AFM and EM will provide information on the quaternary structure and morphology of the assembled aggregates and fibers. FFF, a one-phase matrix-free chromatography, would be used to evaluate the different kinds of species formed during assembly of the collagen. Since FFF is a matrix-free chromatography technique, it can separate different dissolved macromolecules, especially fibers, which can not be separated by other classical chromatographic techniques.

Kinetics of the assembly process will also be investigated with CD and FTIR, which can be performed as a function of time by monitoring changes in the secondary and tertiary structure during the fiber formation. Further, static light scattering, dynamic light scattering and time-lapse AFM allow to monitor protein assembly in real-time.

Fluorescent dyes can also be used to investigate the structural changes associated with protein assembly. The fluorescent properties of some dyes, such as the N-benzyl derivatives of 3-chloro-6-methoxy-9 aminoacridine and amino naphthalene sulfonic acids, change with the polarity of the protein environment. Therefore, labeling of collagen with these dyes are used to study its assembly process.

### Study of the role of metals in the assembly and cross-linking of Collagens

### Role of GGH in DOPA and tyrosine cross-links

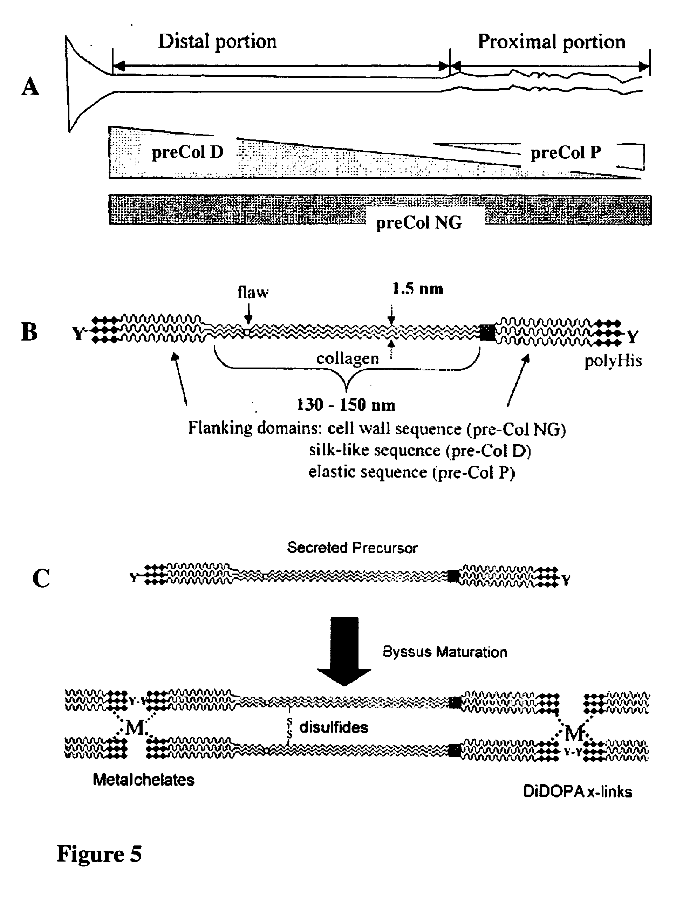

The amino acid sequence GGH has been observed at the carboxylterminus of both preColP and preColD. The tripeptide NH2-Gly-Gly-His-COOH(GGH) mediates cross-linking of associated proteins in solution in the presence of nickel acetate [Ni(OAc)2] and oxidant magnesium monoperoxyphthalate (MMPP) [18, 19]. Further, the peptide provides a favorable coordination environment for the nickel center, and a putative Ni(III) intermediate is thought to abstract an electron from the aromatic ring of an accessible tyrosine, leading to a tyrosyl radical after the loss of a proton (see figure 5). The highly activated radical intermediate couples to a nearby tyrosine leading to a cross-linked adduct.

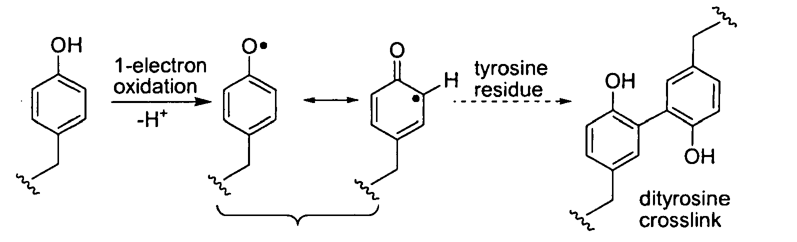

### Mechanism of tyrosine cross-linking

A possible role for the GGH in the carboxylterminus of mussel collagens could be to bind Ni(II) in order to form the active catalyst Ni-GGH. This complex can slowly catalyze aerial oxidation of tyrosine and DOPA to form cross-links. The proximity of the catalyst to tyrosine and/or DOPA would significantly increase the oxidation rates. To test this hypothesis, the GGH sequence could be genetically deleted or modified so that it would not bind nickel. The rate of cross-linking and assembly would be monitored by methods described above.

### Chemical oxidation of tyrosines to form cross-links in Collagen

Visible-light irradiation in the presence of ruthenium(II)tris(bipyridyl) dication [Ru(bpy)₃²⁺] and an electron acceptor such as ammonium persulfate (APS) [18, 20, 21] induces very efficient cross-linking between contacting proteins. This process is highly efficient and the mechanism has been assumed to be similar to that of Ni/GGH/MMPP. The fiber formed from self-assembly of preColP and / or preColD will be subject to irradiation with [Ru(bpy)₃²⁺] in the presence of APS. This should lead to increased cross-linking of tyrosine/DOPA in byssal collagen and lead to fibers with altered mechanical properties, which will be assessed upon physico-chemical characterization as described above.

Further examples and sequences:

DNA sequences of mussel collagen preColP and proColD are provided in the following. The cDNA of both preCol proteins (P and D) were integrated in the pGEM-T cloning vectors. In order to verify the starting material, both cDNAs were completely sequenced and as standard primers T7 and SP6 were used and as internal primers preCol (P or D)-T7/1 and SP6/1, respectively, were used. The obtained DNA sequences showed differences as regards the published versions of both preCols and were compared accordingly.

### preColP (SEQ ID NO:1)

atggttcg gttctcccta gcatcggtac tattactggc agtcaccagc acagctttcg ctggaccagt
tagtgattat ggtggtggtg gaatcaaagt agtaccctac cacggaggcg gaggtggaag
cggcggcggt ggcggtggag gccatggcgg aagcggtatt ggtggtatcg gaggaggatc
atcacatgca catgcccact cttcagcatc tgcccatgtg caccattttg gaccaggtgg
atcttcacat gcatcagctg gttcatcatc ccatgcatcc gcatcccata acggtttagg
aggtggcagt gctcatgcac atagcagttc cagcgccaac gctcattccg gtggattcgg
tggattcggc ggtattggtg gtattggcgg tattggccca ggaggaagtg tcggaggcgg
tattggccca ggaggaagtg tcggaggcgg cattggcggt attggcggta ttggcggcgg
tggtggacca ggcggtaatg gcggtatcgg attcggacca ggattcggag gaggattcgg
accaggttca tctgctagtg gatccggaag tggcagcgca ttcggtggtc caggaggttc
aagcgcaagc gcaaacgcag ctgcacgtgc aaatgcaaat ggtggtggag gattcggtgg
accaggtacc ccaggaaact caggaccacc aggccaaccc ggactaccag gagcaccagg
ccaaccagga cgtccaggaa gtaccccacc aggtcgacca ggaaaccccg gaccaccagg
tcaaccaggt aacccaggac gtccaggctc ttcaggaaga ccaggaggat ccggccaacc
aggaggtcca ggacgtccag gaacccccgg caaaccagga aaccgaggac aaccaggaca
gccaggcggc ccaggacaac caggtcaccc aggagcagga ggacaaccag gacgaaacgg
aaatccagga aaccccggta aaccaggaac accaggtcac ccaggaacag caggatcacg
aggaatgcca ggaaccccag gaaccccagg acaaccagga attccaggca ccgtcggagg
acgaggacca agaggaccag ctggaatcat cggattaatt ggaccaaaag gaaatccagg
agagccagga aatccaggtg caccaggagg cccaggatct acaggaccac aaggaccaca
aggaccagcc ggaggaccag gagcatcagg cggaccagga gacaaaggcg caccaggtac
accaggagga actggaccaa gaggaccaat cggaccatca ggaccatcag gagcaccagg
ggaccaagga ccacaaggag gtagaggaac accaggactc gcaggcaaac caggacctaa
aggactacaa ggatcaaatg gagaagttgg accccaagga ccatctggac ccgcaggacc
acaaggccca caaggaaaga acggtgtcaa aggagcagca ggagatcaag gagctagggg
accagaagga aaagccggac cagctggacc acaaggagaa acaggaccaa aaggaccaac
aggagcacaa ggaccagccg gtccagccgg accatcagga gaacaaggac caggagggga
aagaggaggc cagggaccac aaggagctga aggaccaagt ggaccagcag gaccaagagg
accagcagga tcacaaggac caagtggtga acgcggagaa ccaggagcac caggtaaaaa
aggaccaaat ggagaccgag gaaaccaagg atcaccagga gcaccaggca aaaacggagc
acgaggaaat agaggatcaa gaggaagcaa cggatcaccc ggcagatcag gatcaccagg
aagccgagga aaaccaggac cacaaggacc acatggacca agaggagcaa gaggatcacc
aggacaaaaa ggaccacgtg gagaccaagg agcaccaggt gttattcgta ttgttatcga
tgaccagaga acaggaccag aagttgcaga attcccagga tttggtggat tcggaggagc
ttcagctaac gcagcaagtt cagcaaatgc atttgctggt ggacccggtg gttccgctgg
agcaggttca tcatcaggag ctaacgcaaa cgcaggtgga ttcccattcg gaggaggacc
attcggagga gcaggaggtg gtcccggagc agcaggaggc ccaggaggag caggaggccc
aggaggagta ggaggaggag ttggaggtgg accaggagga gtaggaggtg gagtaggagg
tggaccagga ggagtaggag gtggaccagg aggagcagga ccaggaggag caggaggatt
tggaccagga ggagcaggag gatttggtgg atttggagga ggatctagcg ctggagcatc
atcatcagga tcagcatctg catctaacgg tggaccattc ggagtactca atgtaggacc

cggaggtaga atcggtggtg gaagcgcatc agcatctgca gcatctagag cacatgcaca
cgcttttggt ggtctcggag ggggaagtgc ctcagctggt agtcattcct catctagctc
acactcattt ggcggacacg tattccacag tgtgacccat catggaggtc catcacatgt
ttcaagcgga ggtcacggag gtcatggagg aggtccatac aaacctggat attaa

Due to the degeneration of the genetic code, not every base exchange is leading to an amino acid exchange. Therefore, the DNA sequences were translated into the amino acid sequences and were compared. Here, the alignment of the published sequence (COYNE et al., 1997) [gi:2386675] (variant P38, (COYNE & WAITE, 2000)) with the sequence of preColP obtained by sequencing is shown. The database sequence corresponds to SEQ ID NO: 9, the sequenced preColP sequence is SEQ ID NO:3.

Database 1 MVRFSLASVLLLAVTSTAFAGPVSDYGGGGIKVVPYHGGGGGGGGGGGGG 50
||||||||||||||||||||||||||||||||||||||||||.|||||||
Sequencing 1 MVRFSLASVLLLAVTSTAFAGPVSDYGGGGIKVVPYHGGGGGSGGGGGGG 50

Database 51 HGGSFRNGRHGGIGGIGGGSSHAHAHSSASAHVHHFGPGGSSHASAGSSS 100
||||       |||||||||||||||||||||||||||||||||||||||
Sequencing 51 HGGS-------GIGGIGGGSSHAHAHSSASAHVHHFGPGGSSHASAGSSS 93

Database 101 HASASHNGLGGGSAHAHSSSSANAHSGGFGGFGGIGGIGGIGPGGSVGGG 150
||||||||||||||||||||||||||||||||||||||||||||||||||
Sequencing 94 HASASHNGLGGGSAHAHSSSSANAHSGGFGGFGGIGGIGGIGPGGSVGGG 143

Database 151 IGPGGSVGGGIGGIGGIGGGGGPGGNGGIGFGPGFGGGFGPGSSASGSGS 200
||||||||||||||||||||||||||||||||||||||||||||||||||
Sequencing 144 IGPGGSVGGGIGGIGGIGGGGGPGGNGGIGFGPGFGGGFGPGSSASGSGS 193

Database 201 GSAFGGPGGSSASANAAARANANGGGGFGGPGTPGNSGPPGQPGLPGAPG 250
||||||||||||||||||||||||||||||||||||||||||||||||||
Sequencing 194 GSAFGGPGGSSASANAAARANANGGGGFGGPGTPGNSGPPGQPGLPGAPG 243

Database 251 QPGRPGSTPPGRLGNPGPPGQPGNPGRPGSSGRPGGSGQPGGPGRPGTPG 300
||||||||||||.|||||||||||||||||||||||||||||||||||||
Sequencing 244 QPGRPGSTPPGRPGNPGPPGQPGNPGRPGSSGRPGGSGQPGGPGRPGTPG 293

Database 301 KPGNRGQPGQPGGPGQPGHPGAGGQPGRNGNPGNPGKPGTPGHPGTAGSR 350
||||||||||||||||||||||||||||||||||||||||||||||||||
Sequencing 294 KPGNRGQPGQPGGPGQPGHPGAGGQPGRNGNPGNPGKPGTPGHPGTAGSR 343

Database 351 GMPGTPGTPGQPGIPGTVGGRGPRGPAGIIGLIGPKGNPGEPGNPGAPGG 400
||||||||||||||||||||||||||||||||||||||||||||||||||
Sequencing 344 GMPGTPGTPGQPGIPGTVGGRGPRGPAGIIGLIGPKGNPGEPGNPGAPGG 393

Database 401 PGSTGPQGPQGPAGGPGASGGPGDKGAPGTPGGTGPRGPIGPSGPSGAPG 450
||||||||||||||||||||||||||||||||||||||||||||||||||
Sequencing 394 PGSTGPQGPQGPAGGPGASGGPGDKGAPGTPGGTGPRGPIGPSGPSGAPG 443

Database 451 DQGPQGGRGTPGLAGKPGPKGLQGSNGEVGPQGPSGPAGPQGPQGKNGVK 500
||||||||||||||||||||||||||||||||||||||||||||||||||
Sequencing 444 DQGPQGGRGTPGLAGKPGPKGLQGSNGEVGPQGPSGPAGPQGPQGKNGVK 493

Database 501 GAAGDQGARGPEGKAGPAGPQGETGPKGPTGAQGPAGPAGPSGEQGPGGE 550
||||||||||||||||||||||||||||||||||||||||||||||||||
Sequencing 494 GAAGDQGARGPEGKAGPAGPQGETGPKGPTGAQGPAGPAGPSGEQGPGGE 543

Database 551 RGGQGPQGAEGPSGPAGPRGPAGSQGPSGERGEPGAPGKKGPNGDRGNQG 600
||||||||||||||||||||||||||||||||||||||||||||||||||
Sequencing 544 RGGQGPQGAEGPSGPAGPRGPAGSQGPSGERGEPGAPGKKGPNGDRGNQG 593

Database 601 SPGAPGKNGARGNRGSRGSNGSPGRSGSPGSRGKPGPQGPHGPRGLRGSP 650
|||||||||||||||||||||||||||||||||||||||||||||.||||
Sequencing 594 SPGAPGKNGARGNRGSRGSNGSPGRSGSPGSRGKPGPQGPHGPRGARGSP 643

Database 651 GQKGPRGDQGAPGVIRIVIDDQRTGPEVAEFPGFGGFGGASANAASSANA 700
||||||||||||||||||||||||||||||||||||||||||||||||||
Sequencing 644 GQKGPRGDQGAPGVIRIVIDDQRTGPEVAEFPGFGGFGGASANAASSANA 693

Database 701 FAGGPGGSAGAGSSSGANANAGGF-----PFGGAGGGPGAAGGPGGAGGP 745
||||||||||||||||||||||||     |||||||||||||||||||||
Sequencing 694 FAGGPGGSAGAGSSSGANANAGGFPFGGGPFGGAGGGPGAAGGPGGAGGP 743

Database 746 GGVGGGVGGGPGGVGGGVGGGPGGVGGGPGGAGPGGAGGFGPGGAGGFGG 795
||||||||||||||||||||||||||||||||||||||||||||||||||
Sequencing 744 GGVGGGVGGGPGGVGGGVGGGPGGVGGGPGGAGPGGAGGFGPGGAGGFGG 793

Database 796 FGGGSSAGASSSGSASASNGGPFGVLNVGPGGRIGGGSASASAASRAHAH 845
||||||||||||||||||||||||||||||||||||||||||||||||||
Sequencing 794 FGGGSSAGASSSGSASASNGGPFGVLNVGPGGRIGGGSASASAASRAHAH 843

Database 846 AFGGLGGGSASAGSHSSSSSHSFGGHVFHSVTHHGGPSHVSSGGHGGHGG 895
||||||||||||||||||||||||||||||||||||||||||||||||||
Sequencing 844 AFGGLGGGSASAGSHSSSSSHSFGGHVFHSVTHHGGPSHVSSGGHGGHGG 893

Database   896 GPYKPGY 902
               |||||||
Sequencing 894 GPYKPGY 900

COYNE & WAITE already showed the existence of different preColP variants (P22, P33 and P38) in certain partial regions of their cDNA sequence (COYNE & WAITE, 2000). If these short, known sequence regions of variant P22 are compared with the present DNA sequence of preColP, a matching of 100% is achieved.

### preColD (SEQ ID NO:2)

atggtcta caaactcctg accgtgtgtc ttgtagcatc tcttctagag atttgcttag
ctgactataa cggcaacaaa cagtatggcg gcagatacgg caacagatac ggaaacggtt
taggaggcgg taatggtggt gcaggagccg tagcccatgc ccatgcccat gcccatgcca
gtgccggagc aaacggaaga gcaagagcac atgcacgagc cttggcccat gcacatgccg
gtggtggcgc tgcacatgga cacccaggat tcccagttgg tggtagcgca agcgcagccg
cacgagcagc agcacgagca tcagcaggag gattaggtgg attcggatca gcagcagcca
atgcagcagc agcagcaaga gcaggagcag gatttggtgg attcggtgga ttaggaggat
tcggaggact cggaggagtt ggcggtccag gtcaaccagg acatgccggt aaacacggaa
ccgcaggagc agcaggcaaa gcaggacgtc caggaccatg tggagataga ggggcaccag
gagtaccagg caaacaagga ccagtaggag gacaaggacc agcaggacca cgaggaccac
gaggagatga aggaccagtt ggaccaaagg gcgaaccagg agcaagagga gctgatggta
aaccaggaga caaaggacct gatggagaaa ccggaccaca aggaccagct ggaccaaagg
gacaagtagg agaccaaggc aaaccaggag caaagggaga aaccggagat caaggagcac
gaggtgaagc aggaaaggcc ggcgaacaag gaccaggagg catccaagga ccaaagggac
cagtaggagg acaaggacca gcaggaccag ccggaccact cggaccacaa ggaccaatgg
gtgaacgagg accacaagga ccaacaggat cagaaggacc agttggagca ccaggaccaa
agggatcagt cggagaccaa ggagcacaag gagaccaagg agcaactggc gctgatggca
aaaagggaga accaggagag agaggacaac aaggagcagc aggaccagtc ggccgaccag
gaccaagagg agatagagga gcaaagggaa ttcaaggaag ccgaggacga ccaggtggta
tgggtagacg aggaaaccgt ggatcccaag gagcagtagg accacgagga gaaactggcc
cagacggtaa ccaaggacaa cgtggagaac aaggagcacc aggagttatc acccttgtca
ttgaagacct cagaacagcc ggagtagaaa gccccgtaga aacctttgac gcaggagcag
gaaccggtgg accagcacca ggagtaggag cagcagcaac agcaggagca tttgcaggag
caggaccagg aggagctaat gcaggaggaa acgcagccgc aggagcagga ccaggagtag
gaccaggagg actcggagga ctaggaggac ttggtgcagg tggactcgga ggtggactcg
gcggtggact cggaggatta ggaggagcag gaggtttagg tggtggactc ggaggattag
gaggaggttt aggtggtgga ctcggaggtt taggaggtgg agcaggagga gcaggaggcg
caggagcagg aggaaacggt ggagcaggag caggaggagc aggaggaaac ggtggaggat
cagccgcagc acgagcagca gcacaagcag cagcagcagc aggaggaaac ggtggagcag
cacaagcagc agcacaagca gcagcatcag cagcagcaaa ttcaggactt ggagcaggag
cagcaagagc agcagcatca gcagccgcta gagcaaccgt agcaggacat ggaagtggaa
ccgccgcagc agcagccaac gcagccgcac aagcacatgc agcaacacga ggacaaggag
gatcacacgc acacgctgcc gccgcagctc acgcagccgc aagtagcgta atccatggtg
gtgactatca cggaaacgat gccggctatc acaaaccagg atattaa

In the following, the alignment of the published sequence (QIN et al., 1997) [gi:2772914] with the overall sequence obtained by sequencing of preColD is shown. The DNA sequences were translated into the protein sequence. It is noted that the database sequence is SEQ ID NO: 8 and the sequence obtained by sequencing is SEQ ID NO: 4.

Database 1 MVYKLLTVCLVASLLEICLADYNGNKQYGGRYGNRYGNGLGGGNGGAGAV 50
||||||||||||||||||||||||||||||||||||||||||||||||||
Sequencing 1 MVYKLLTVCLVASLLEICLADYNGNKQYGGRYGNRYGNGLGGGNGGAGAV 50

Database 51 AHAHAHAHASAGANGRARAHARALAHAHAGGGAAHGHPGFPVGGSASAAA 100
||||||||||||||||||||||||||||||||||||||||||||||||||
Sequencing 51 AHAHAHAHASAGANGRARAHARALAHAHAGGGAAHGHPGFPVGGSASAAA 100

Database 101 RAAARASAGGLGGFGSAAANAAAAARAGAGFGGFGGLGGFGGLGGVGGPG 150
||||||||||||||||||||||||||||||||||||||||||||||||||
Sequencing 101 RAAARASAGGLGGFGSAAANAAAAARAGAGFGGFGGLGGFGGLGGVGGPG 150

Database 151 QPGGPGGPGGPGGPGGPGMPGGPGGPSGPGTGGPGQPGGPGGPGGPGGPG 200
|
Sequencing 151 Q------------------------------------------------- 151

Database 201 GPSMPGGPGGPGGPGMPGGPGGPGGPGGAGGIPGMTGPAGPPGPAGPQGP 250
Sequencing 151 -------------------------------------------------- 151

Database 251 EGEQGPRGRTPAGTPGPPGNPGEPGQGGAPGAPGAPGHAGKHGTAGAAGK 300
||||||||||||||
Sequencing 152 ------------------------------------PGHAGKHGTAGAAGK 166

Database 301 AGRPGPXGQAGASGSSGQHGASGAPGRPGNPGSTGRPGATGDPGRPGATG 350
||||
Sequencing 167 AGRP---------------------------------------------- 170

Database 351 TTGRPGPSGAPGNPGAPGALGAPGPRGSPGFVGLPGPRGSPGEPGNQGPI 400
Sequencing 170 -------------------------------------------------- 170

Database 401 GGPGYPGPRGPQGPDGAMGPQGPCGDRGAPGVPGKQGPVGGQGPAGPRGP 450
|||||||||||||||||||||||||||||
Sequencing 171 ---------------------GPCGDRGAPGVPGKQGPVGGQGPAGPRGP 199

Database 451 RGDEGPVGPKGEPGARGADGKPGDKGPDGETGPQGPAGPKGQVGDQGKPG 500
||||||||||||||||||||||||||||||||||||||||||||||||||
Sequencing 200 RGDEGPVGPKGEPGARGADGKPGDKGPDGETGPQGPAGPKGQVGDQGKPG 249

Database 501 AKGETGDQGARGEAGKAGEQGPGGIQGPKGPVGGQGPAGPAGPLGPQGPM 550
||||||||||||||||||||||||||||||||||||||||||||||||||

Sequencing 250 AKGETGDQGARGEAGKAGEQGPGGIQGPKGPVGGQGPAGPAGPLGPQGPM 299

Database 551 GERGPQGPTGSEGPVGAPGPKGSVGDQGAQGDQGATGADGKKGEPGERGQ 600
||||||||||||||||||||||||||||||||||||||||||||||||||
Sequencing 300 GERGPQGPTGSEGPVGAPGPKGSVGDQGAQGDQGATGADGKKGEPGERGQ 349

Database 601 QGAAGPVGRPGPRGDRGAKGIQGSRGRPGGMGRRGNRGSQGAVGPRGETG 650
||||||||||||||||||||||||||||||||||||||||||||||||||
Sequencing 350 QGAAGPVGRPGPRGDRGAKGIQGSRGRPGGMGRRGNRGSQGAVGPRGETG 399

Database 651 PDGNQGQRGEQGAPGVITLVIEDLRTAGVESPVETFDAGAGTGGPAPGVG 700
||||||||||||||||||||||||||||||||||||||||||||||||||
Sequencing 400 PDGNQGQRGEQGAPGVITLVIEDLRTAGVESPVETFDAGAGTGGPAPGVG 449

Database 701 AAATAGAFAGAGPGGANAGGNAAAGAGPGVGPGGLGGLGGLGAGGLGGGL 750
||||||||||||||||||||||||||||||||||||||||||||||||||
Sequencing 450 AAATAGAFAGAGPGGANAGGNAAAGAGPGVGPGGLGGLGGLGAGGLGGGL 499

Database 751 GGGLGGLGGAGGLGGGLGGLGGGLGGGLGGLG---GGAGGAGAGGNGGAG 797
||||||||||||||||||||||||||||||||   |||||||||||||||
Sequencing 500 GGGLGGLGGAGGLGGGLGGLGGGLGGGLGGLGGGAGGAGGAGAGGNGGAG 549

Database 798 AGGAGGNGGGSAAARAAAQAAAAAGGNGGAAQAAAQAAASAAANSGLGAG 847
||||||||||||||||||||||||||||||||||||||||||||||||||
Sequencing 550 AGGAGGNGGGSAAARAAAQAAAAAGGNGGAAQAAAQAAASAAANSGLGAG 599

Database 848 AARAAASAAARATVTGHGSGTAAAAANAAAQAHAATRGQGGSHAHAAAAA 897
||||||||||||||.|||||||||||||||||||||||||||||||||||
Sequencing 600 AARAAASAAARATVAGHGSGTAAAAANAAAQAHAATRGQGGSHAHAAAAA 649

Database 898 HAAASSVIHGGDYHGNDAGYHKPGY 922
|||||||||||||||||||||||||
Sequencing 650 HAAASSVIHGGDYHGNDAGYHKPGY 674

There are significant differences in both sequences: The preColD sequence used is by 250 amino acids shorter than the published sequence. The major part of the amino acids in the collagen domain is missing. Therefore, the presently disclosed preColD gene is an up to now unpublished and unknown version of the preColD gene. It is noted that the truncation of the collagen domain increases the amount of silk fibroin domains in the whole protein and therefore, the behavior of the overall protein will be different.

### EXPRESSION CONSTRUCT of P4H

In the following, the DNA sequence after expression plasmid for P4H in the region of *SacII* to *ApaI* is shown as double strand. The beginning and the end of MFa/P4H fusion constructs are both printed. The used restriction sites are underligned.

### SEQ ID NO: 7

1 ccgcggtcat tacagttcat ctttcacagc tttctgatca tcgtcttcct ccatgtctgg
1 ggcgccagta atgtcaagta gaaagtgtcg aaagactagt agcagaagga ggtacagacc
SacII 2xStop

61 ctcctctgct tcttccaggt cctcgagatc gtcatcatcc cctgccccat cctggccacc
61 gaggagacga agaaggtcca ggagctctag cagtagtagg ggacggggta ggaccggtgg

121 cgagaggtcc ttaaagaatt ttggtaggtc gcacgcaagg ggcaacatta gttactggca
121 gctctccagg aatttcttaa aaccatccag cgtgcgttcc ccgttgtaat caatgaccgt

181 cctgtcggca ctggcaggaa agaacttgag tgtggggaag ctgtgcactt tgacggcctc
181 ggacagccgt gaccgtcctt tcttgaactc acaccccttc gacacgtgaa actgccggag

241 cacctcgttg gcagtcgagt ccatcttggc gatgacgatg ttctcatggt ccttgtacgt
241 gtggagcaac cgtcagctca ggtagaaccg ctactgctac aagagtacca ggaacatgca

301 ctctcccagt ttatcccaaa tgggagccaa ctgtttgcag tgaccacacc atggggcata
301 gagagggtca aatagggttt accctcggtt gacaaacgtc actggtgtgg taccccgtat

361 gaactccaca aagacgtttt ttttctcatc aaaagccacg tcttcaaagt tcttcccaac
361 cttgaggtgt ttctgcaaaa aaaagagtag ttttcggtgc agaagtttca agaagggttg

421 aagcaccttg acaggctgct tgtcccagtc ctccggcagc tcctggctca tcaggtgggg
421 ttcgtggaac tgtccgacga acagggtcag gaggccgtcg aggaccgagt agtccacccc

481 cttgattttg ccctccagga agcggtggca gaactctgtg atcctctctg ccgtcagctc
481 gaactaaaac gggaggtcct tcgccaccgt cttgagacac taggagagac ggcagtcgag

541 ctccgattcg ggcttgtact tggtcatctc ctcctccagg gtgatgaggc gcacggccgg
541 gaggctaagc ccgaacatga accagtagag gaggaggtcc cactactccg cgtgccggcc

601 gcactcttcc ttcttcaggc caaagaactc gaggatgcgc tggttgtcgg tgtggtcgct
601 cgtgagaagg aagaagtccg gtttcttgag ctcctacgcg accaacagcc acaccagcga

661 gtcgatgaag atgaacagga tcttgccctt gaagctctcg gctgctgttt tgaagttgct
661 cagctacttc tacttgtcct agaacgggaa cttcgagagc cgacgacaaa acttcaacga

721 cagtttgccg tcatagtcag acacactctt gggcaagaac agcaggatgt gagtcttgat
721 gtcaaacggc agtatcagtc tgtgtgagaa cccgttcttg tcgtcctaca ctcagaacta

781 ttcacctcca aaaatcttcg gggctgtctg ctcggtgaac tcgatgacaa ggggcagctg
781 aagtggaggt ttttagaagc cccgacagac gagccacttg agctactgtt ccccgtcgac
841 gttgtgtttg ataaagtcca gcaggttctc cttggtgacc tccccttcaa agttgttccg
841 caacacaaac tatttcaggt cgtccaagag gaaccactgg aggggaagtt tcaacaaggc

901 gccttcatca aacttcttaa agaggacaac cccatctttg tcgagctggt atttggagaa
901 cggaagtagt ttgaagaatt tctcctgttg gggtagaaac agctcgacca taaacctctt

961 cacgtcactg ttggaagtga tcccaaatgg tatgtcatcg atggcctctg ctgcctgcaa
961 gtgcagtgac aaccttcact agggtttacc atacagtagc taccggagac gacggacgtt

1021 aaactgcttg gcagagtccg actccacgtc cttgaagaag ccgatgacag ccacctcgct
1021 tttgacgaac cgtctcaggc tgaggtgcag gaacttcttc ggctactgtc ggtggagcga

1081 ggactccacc aaggactctg cagctgcgcc gtcaggcagg gtggtggcag ccgggcccgt
1081 cctgaggtgg ttcctgagac gtcgacgcgg cagtccgtcc caccaccgtc ggcccgggca
ApaI

1141 gcgcttcttc agccagttca cgatgtcatc agcctctctg ccagctgtat attccttggg
1141 cgcgaagaag tcggtcaagt gctacagtag tcggagagac ggtcgacata taaggaaccc

1201 ggaagccgtg tctccattcc tgaagaactt gatggtggga tagccgcgca cgccgtactg
1201 ccttcggcac agaggtaagg acttcttgaa ctaccaccct atcggcgcgt gcggcatgac

1261 ctgggccagg tcagactcct ccgtggcgtc caccttggcc aacctgatct cggaaccttc
1261 gacccggtcc agtctgagga ggcaccgcag gtggaaccgg ttggactaga gccttggaag

1321 tgccttcagc ttcccagcgg ctttggcata ctcaggggcc agagccttgc agtggccaca
1321 acggaagtcg aagggtcgcc gaaaccgtat gagtccccgg tctcggaacg tcaccggtgt

1381 ggttccccgt atcttgaggt ggtcgtccat gaacacccgg cggtcgcgga ggcgcttcaa
1381 ccaaggggca tagaactcca ccagcaggta cttgtgggcc gccagcgcct ccgcgaagtt

1441 gcttttccgc agcaccagga cgtggtcctc ctcctccgga gcgtcagcta atgcggagga
1441 cgaaaaggcg tcgtggtcct gcaccaggag gaggaggcct cgcagtcgat tacgcctcct
BspEI

1501 tgctgcgaat aaaactgcag taaaaattga aggaaatctc atggatccgg ggttttttct
1501 acgacgctta ttttgacgtc atttttaact tcctttagag tacctaggcc ccaaaaaaga
Start BamHI

1561 ccttgacgtt aaagtataga ggtatattaa caattttttg ttgatacttt tattacattt
1561 ggaactgcaa tttcatatct ccatataatt gttaaaaaac aactatgaaa ataatgtaaa

1621 gaataagaag taatacaaac cgaaaatgtt gaaagtatta gttaaagtgg ttatgcagtt
1621 cttattcttc attatgtttg gcttttacaa ctttcataat caatttcacc aatacgtcaa

1681 tttgcattta tatatctgtt aatagatcaa aaatcatcgc ttcgctgatt aattacccca
1681 aaacgtaaat atatagacaa ttatctagtt tttagtagcg aagcgactaa ttaatggggt

1741 gaaataaggc taaaaaacta atcgcattat catcctatgg ttgttaattt gattcgttca
1741 ctttattccg attttttgat tagcgtaata gtaggatacc aacaattaaa ctaagcaagt

1801 tttgaaggtt tgtggggcca ggttactgcc aatttttcct cttcataacc ataaaagcta
1801 aaacttccaa acaccccggt ccaatgacgg ttaaaaagga gaagtattgg tattttcgat

1861 gtattgtaga atctttattg ttcggagcag tgcggcgcga ggcacatctg cgtttcagga
1861 cataacatct tagaaataac aagcctcgtc acgccgcgct ccgtgtagac gcaaagtcct

1921 acgcgaccgg tgaagacgag gacgcacgga ggagagtctt ccttcggagg gctgtcaccc
1921 tgcgctggcc acttctgctc ctgcgtgcct cctctcagaa ggaagcctcc cgacagtggg

1981 gctcggcggc ttctaatccg tacttcaata tagcaatgag cagttaagcg tattactgaa
1981 cgagccgccg aagattaggc atgaagttat atcgttactc gtcaattcgc ataatgactt

2041 agttccaaag agaaggtttt tttaggctaa gataatgggg ctctttacat ttccacaaca
2041 tcaaggtttc tcttccaaaa aaatccgatt ctattacccc gagaaatgta aaggtgttgt

2101 tataagtaag attagatatg gatatgtata tggatatgta tatggtggta atgccatgta
2101 atattcattc taatctatac ctatacatat acctatacat ataccaccat tacggtacat

2161 atatgattat taaacttctt tgcgtccatc caaaaaaaaa gtaagaattt ttgaaaattc
2161 tatactaata atttgaagaa acgcaggtag gttttttttt cattcttaaa aacttttaag

2221 aaggaattcg atatcaagct tatcgatacc gtcgacatga gatttccttc aatttttact
2221 ttccttaagc tatagttcga atagctatgg cagctgtact ctaaaggaag ttaaaaatga
EcoRI SalI Start

2281 gcagttttat tcgcagcatc ctccgcgcta gctcatccag gcttttttac ttcaattggt
2281 cgtcaaaata agcgtcgtag gaggcgcgat cgagtaggtc cgaaaaaatg aagttaacca
NheI

2341 cagatgactg atttgatcca tactgagaaa gatctggtga cttctctgaa agattatatt
2341 gtctactgac taaactaggt atgactcttt ctagaccact gaagagactt tctaatataa

2401 aaggcagaag aggacaagtt agaacaaata aaaaaatggg cagagaagtt agatcggcta
2401 ttccgtcttc tcctgttcaa tcttgtttat ttttttaccc gtctcttcaa tctagccgat

2461 actagtacag cgacaaaaga tccagaagga tttgttgggc atccagtaaa tgcattcaaa
2461 tgatcatgtc gctgttttct aggtcttcct aaacaacccg taggtcattt acgtaagttt

2521 ttaatgaaac gtctgaatac tgagtggagt gagttggaga atctggtcct taaggatatg
2521 aattactttg cagacttatg actcacctca ctcaacctct tagaccagga attcctatac

2581 tcagatggct ttatctctaa cctaaccatt cagagaccag tactttctaa tgatgaagat
2581 agtctaccga aatagagatt ggattggtaa gtctctggtc atgaaagatt actacttcta

2641 caggttgggg cagccaaagc tctgttacgt ctccaggata cctacaattt ggatacagat
2641 gtccaacccc gtcggtttcg agacaatgca gaggtcctat ggatgttaaa cctatgtcta

2701 accatctcaa agggtaatct tccaggagtg aaacacaaat cttttctaac ggctgaggac
2701 tggtagagtt tcccattaga aggtcctcac tttgtgttta gaaaagattg ccgactcctg

2761 tgctttgagt tgggcaaagt ggcctataca gaagcagatt attaccatac ggaactgtgg
2761 acgaaactca acccgtttca ccggatatgt cttcgtctaa taatggtatg ccttgacacc

2821 atggaacaag ccctaaggca actggatgaa ggcgagattt ctaccataga taaagtctct
2821 taccttgttc gggattccgt tgacctactt ccgctctaaa gatggtatct atttcagaga

2881 gttctagatt atttgagcta tgcggtatat cagcagggag acctggataa ggcacttttg
2881 caagatctaa taaactcgat acgccatata gtcgtccctc tggacctatt ccgtgaaaac

2941 ctcacaaaga agcttcttga actagatcct gaacatcaga gagctaatgg taacttaaaa
2941 gagtgtttct tcgaagaact tgatctagga cttgtagtct ctcgattacc attgaatttt

3001 tattttgagt atataatggc taaagaaaaa gatgtcaata agtctgcttc agatgaccaa
3001 ataaaactca tatattaccg atttcttttt ctacagttat tcagacgaag tctactggtt

3061 tctgatcaga aaactacacc aaagaaaaaa ggggttgctg tggattacct gccagagaga
3061 agactagtct tttgatgtgg tttctttttt ccccaacgac acctaatgga cggtctctct

3121 cagaagtacg aaatgctgtg ccgtggggag ggtatcaaaa tgacccctcg gagacagaaa
3121 gtcttcatgc tttacgacac ggcacccctc ccatagtttt actggggagc ctctgtcttt

3181 aaactctttt gccgctacca tgatggaaac cgtaatccta aatttattct ggctccagct
3181 tttgagaaaa cggcgatggt actacctttg gcattaggat ttaaataaga ccgaggtcga

3241 aaacaggagg atgaatggga caagcctcgt attattcgct tccatgatat tatttctgat
3241 tttgtcctcc tacttaccct gttcggagca taataagcga aggtactata ataaagacta

3301 gcagaaattg aaatcgtcaa agacctagca aaaccaaggc tgagccgagc tacagtacat
3301 cgtctttaac tttagcagtt tctggatcgt tttggttccg actcggctcg atgtcatgta

3361 gaccctgaga ctggaaaatt gaccacagca cagtacagag tatctaagag tgcctggctc
3361 ctgggactct gaccttttaa ctggtgtcgt gtcatgtctc atagattctc acggaccgag

3421 tctggctatg aaaatcctgt ggtgtctcga attaatatga gaatacaaga tctaacagga
3421 agaccgatac ttttaggaca ccacagagct taattatact cttatgttct agattgtcct

3481 ctagatgttt ccacagcaga ggaattacag gtagcaaatt atggagttgg aggacagtat
3481 gatctacaaa ggtgtcgtct ccttaatgtc catcgtttaa tacctcaacc tcctgtcata

3541 gaaccccatt ttgactttgc acggaaagat gagccagatg ctttcaaaga gctggggaca
3541 cttggggtaa aactgaaacg tgcctttcta ctcggtctac gaaagtttct cgacccctgt

3601 ggaaatagaa ttgctacatg gctgttttat atgagtgatg tgtctgcagg aggagccact
3601 cctttatctt aacgatgtac cgacaaaata tactcactac acagacgtcc tcctcggtga

3661 gtttttcctg aagttggagc tagtgtttgg cccaaaaaag gaactgctgt tttctggtat
3661 caaaaaggac ttcaacctcg atcacaaacc gggttttttc cttgacgaca aaagaccata

3721 aatctgtttg ccagtggaga aggagattat agtacacggc atgcagcctg tccagtgcta
3721 ttagacaaac ggtcacctct tcctctaata tcatgtgccg tacgtcggac aggtcacgat

3781 gttggcaaca aatgggtatc caataaatgg ctccatgaac gtggacaaga atttcgaaga
3781 caaccgttgt ttacccatag gttatttacc gaggtacttg cacctgttct taaagcttct

3841 ccttgtacgt tgtcagaatt ggaatgatga gggccc
3841 ggaacatgca acagtcttaa ccttactact cccggg
2xStop ApaI

### Protein sequences

### MFa - P4HA (SEQ ID NO:5)

MRFPSIFTAV LFAASSALAH PGFFTSIGQM TDLIHTEKDL VTSLKDYIKA EEDKLEQIKK
WAEKLDRLTS TATKDPEGFV GHPVNAFKLM KRLNTEWSEL ENLVLKDMSD GFISNLTIQR
PVLSNDEDQV GAAKALLRLQ DTYNLDTDTI SKGNLPGVKH KSFLTAEDCF ELGKVAYTEA

DYYHTELWME QALRQLDEGE ISTIDKVSVL DYLSYAVYQQ GDLDKALLLT KKLLELDPEH
QRANGNLKYF EYIMAKEKDV NKSASDDQSD QKTTPKKKGV AVDYLPERQK YEMLCRGEGI
KMTPRRQKKL FCRYHDGNRN PKFILAPAKQ EDEWDKPRII RFHDIISDAE IEIVKDLAKP
RLSRATVHDP ETGKLTTAQY RVSKSAWLSG YENPVVSRIN MRIQDLTGLD VSTAEELQVA
NYGVGGQYEP HFDFARKDEP DAFKELGTGN RIATWLFYMS DVSAGGATVF PEVGASVWPK
KGTAVFWYNL FASGEGDYST RHAACPVLVG NKWVSNKWLH ERGQEFRRPC TLSELE

### MFa - P4HB (SEQ ID NO:6)

MRFPSIFTAV LFAASSALAD APEEEDHVLV LRKSNFAEAL AAHKYLLVEF YAPWCGHCKA
LAPEYAKAAG KLKAEGSEIR LAKVDATEES DLAQQYGVRG YPTIKFFRNG DTASPKEYTA
GREADDIVNW LKKRTGPAAT TLPDGAAAES LVESSEVAVI GFFKDVESDS AKQFLQAAEA
IDDIPFGITS NSDVFSKYQL DKDGVVLFKK FDEGRNNFEG EVTKENLLDF IKHNQLPLVI
EFTEQTAPKI FGGEIKTHIL LFLPKSVSDY DGKLSNFKTA AESFKGKILF IFIDSDHTDN
QRILEFFGLK KEECPAVRLI TLEEEMTKYK PESEELTAER ITEFCHRFLE GKIKPHLMSQ
ELPEDWDKQP VKVLVGKNFE DVAFDEKKNV FVEFYAPWCG HCKQLAPIWD KLGETYKDHE
NIVIAKMDST ANEVEAVKVH SFPTLKFFPA SADRTVIDYN GERTLDGFKK FLESGGQDGA
GDDDDLEDLE EAEEPDMEED DDQKAVKDEL

### Sequence of MFa (SEQ ID NO: 10)

MRFPSIFTAV LFAASSALA

### References:

1. Yonge, M. (1962). On the significance of the byssus in the bivalvia and its effects in evolution. J. Mar. Biol. Ass. U. K. 42, 113-125.
2. Qin, X.-X., Coyne, K.J., and Waite, J.H. (1997). Tough tendons. Mussel byssus has collagen with silk-like domains. Journal of Biological Chemistry 272, 32623-32627.
3. Waite, J.H. (1992). Results Probl. Cell Differ. 19, 27.
4. Qin, X.-X., and Waite, J.H. (1995). Exotic Collagen Gradients in the Byssus of ht eMussel Mytilus Edulia. The Journal of Experimental Biology 198, 633-644.
5. Vaccaro, E., and Waite, J.H. (2001). Yield and Post-Yield Behavior of Mussel Byssal Thread: A Self-Healing Biomolecular Material. Biomacromolecules 2, 906-911.
6. Coyne, K.J., Qin, X.-X., and Waite, J.H. (1997). Extensible collagen in mussel byssus: a natural block copolymer. Science (Washington, D. C.) 277, 1830-1832.
7. Waite, J.H., Qin, X.-X., and Coyne, K.J. (1998). The peculiar collagens of mussel byssus. Matrix Biology 17, 93-106.
8. Coombs, T.L., and Keller, P.J. (1981). Mytilus byssal threads as an environmental marker for metal ions. Aquat. Toxicol. 1981, 291-300.
9. Swann, C.P., Adewole, T., and Waite, J.H. (1998). Preferential manganese accumulation in dreissenid byssal threads. Comparative Biochemistry and Physiology, Part B: Biochemistry & Molecular Biology 119B, 755-759.
10. Taylor, S.W., Chase, D.B., Emptage, M.H., Nelson, M.J., and Waite, J.H. (1996). Ferric Ion Complexes of a DOPA-Containing Adhesive Protein from Mytilus edulis. Inorganic Chemistry 35, 7572-7577.
11. Sun, C., Vaccaro, E., and Waite, J.H. (2001). Oxidative stress and the mechanical properties of naturally occurring chimeric collagen-containing fibers. Biophysical Journal 81, 3590-3595.
12. Myllyharju, J., Nokelainen, M., Vuorela, A., and Kivirikko, K.I. (2000). Expression of recombinant human I-III collagens in the yeast Pichia pastoris. Biochem. Soc. Trans. 28, 353-357.
13. Prockop, D.J., and Kivirikko, K.I. (1995). Annu. Rev. Biochem. 64, 403-434.
14. Olsen, D.R., Leigh, S.D., Chang, R., McMullin, H., Ong, W., Ernest, T., Chisholm, G., Birk, D.E., Berg, R.A., Hitzeman, R.A., and Toman, P.D. (2001). Production of Human Type 1 Collagen in Yeast Reveals Unexpected New Insights into Molecular assembly of Collagen Trimers. J. Biol. Chem. 276, 24038-24043.
15. Scheibel, T. (2004). Spider silks: recombinant synthesis, assembly, spinning, and engineering of synthetic proteins. Microbial Cell Factories 3, No pp. given.
16. Huemmerich, D., Scheibel, T., Vollrath, F., Cohen, S., Gat, U., and Ittah, S. (2004). Novel Assembly Properties of Recombinant Spider Dragline Silk Proteins. Current Biology 14, 2070-2074.
17. Huemmerich, D., Helsen, C.W., Quedzuweit, S., Oschmann, J., Rudolph, R., and Scheibel, T. (2004). Primary Structure Elements of Spider Dragline Silks and Their Contribution to Protein Solubility. Biochemistry 43, 13604-13612.
18. Brown, K.C., and Kodadek, T. (2001). Protein cross-linking mediated by metal ion complexes. Metal Ions in Biological Systems 38, 351-384.
19. Fancy, D.A., Denison, C., Kim, K., Xie, Y., Holdeman, T., Amini, F., and Kodadek, T. (2000). Scope, limitations and mechanistic aspects of the photo-induced cross-linking of proteins by water-soluble metal complexes. Chemistry & Biology 7, 697-708.
20. Burdine, L., Gillette, T.G., Lin, H.-J., and Kodadek, T. (2004). Periodate-Triggered Cross-Linking of DOPA-Containing Peptide-Protein Complexes. Journal of the American Chemical Society 126, 11442-11443.
21. Kim, K., Fancy, D.A., Carney, D., and Kodadek, T. (1999). Photoinduced Protein Cross-Linking Mediated by Palladium Porphyrins. Journal of the American Chemical Society 121, 11896-11897.

### Further references:

Brake, A.J. (1990) Alpha-factor leader-directed secretion of heterologous proteins from yeast. Methods Enzymol. 185: 408-21
Bulleid, N.J., John, D.C. & Kadler, K.E. (2000) Recombinant expression systems for the production of collagen. Biochem. Soc. Trans. 28: 350-3
Coyne, K.J. & Waite, J.H. (2000) In search of molecular dovetails in mussel byssus: from the threads to the stem. J. Exp. Biol. 203: 1425-31
Keizer-Gunnink, I., Vuorela, A., Myllyharju, J., Pihlajaniemi, T., Kivirikko, K.I. & Veenhuis, M. (2000) Accumulation of properly folded human type III procollagen molecules in specific intracellular membranous compartments in the yeast Pichia pastoris. Matrix Biol. 19: 29-36
Lucas, J.M., Vaccaro, E. & Waite, J.H. (2002) A molecular, morphometric and mechanical comparison of the structural elements of byssus from Mytilus edulis and Mytilus galloprovincialis. J. Exp. Biol. 205: 1807-1
Mascolo, J.M. & Waite, J.H. (1986) Protein gradients in byssal threads of some marine bivalve molluscs. J. Exp. Zool. 240: 1-7
Qin, X.X. & Waite, J.H. (1998) A potential mediator of collagenous block copolymer gradients in mussel byssal threads. Proc. Natl. Acad. Sci. USA 95: 10517-22
Sikorski R.S. & Hieter P. (1989) A system of shuttle vectors and yeast host strains designed for efficient manipulation of DNA in Saccharomyces cerevisiae. Genetics 122, 19-27
Toman, P.D., Chisholm, G., McMullin, H., Giere, L.M., Olsen, D.R., Kovach, R.J., Leigh, S.D., Fong, B.E., Chang, R., Daniels, G.A., Berg, R.A. & Hitzeman, R.A. (2000) Production of recombinant human type I procollagen trimers using a four-gene expression system in the yeast Saccharomyces cerevisiae. J. Biol. Chem. 275: 23303-9
Vaughn, P.R., Galanis, M., Richards, K.M., Tebb, T.A., Ramshaw, J.A. & Werkmeister, J.A. (1998) Production of recombinant hydroxylated human type III collagen fragment in Saccharomyces cerevisiae. DNA Cell Biol. 17: 511-8
Vuorela, A., Myllyharju, J., Nissi, R., Pihlajaniemi, T. & Kivirikko, K.I. (1997) Assembly of human prolyl 4-hydroxylase and type III collagen in the yeast pichia pastoris: formation of a stable enzyme tetramer requires coexpression with collagen and assembly of a stable collagen requires coexpression with prolyl 4-hydroxylase. EMBO J. 16: 6702-12
Waite, J.H., Vaccaro, E., Sun, C. & Lucas, J.M. (2002) Elastomeric gradients: a hedge against stress concentration in marine holdfasts? Philos. Trans. R. Soc. Lond B Biol. Sci. 357: 143-53

## Claims

1. A recombinant mussel byssus protein comprising or consisting of one or more fragments of a collagen domain flanked by elastin or silk fibroin.

2. The recombinant mussel byssus protein of claim 1, wherein the fragments are derived from *Mytilus sp.,* preferably *M. edulis, M. galloprovincialis, M. californians,* or *Geukeria demissa.*

3. The recombinant mussel byssus protein of claim 1 or claim 2, comprising or consisting of one or more of the fragments preColP and/or preColD or variants thereof.

4. The recombinant protein of one or more of the preceding claims, which comprises or consists of the amino acid sequence of SEQ ID NO: 3 and/or 4 or variants thereof.

5. The recombinant protein of one or more of claims 1-4, wherein the signal sequence of the respective amino acid sequence is replaced by a host, preferably yeast, specific signal sequence, preferably by mating factor alpha 1 (MFa) of *S. cerevisiae* (SEQ ID NO: 10).

6. An isolated nucleic acid encoding the recombinant protein of one or more of the preceding claims.

7. An isolated nucleic acid encoding the recombinant protein of one or more of claims 1 - 5 comprising or consisting of fragments of SEQ ID NO: 1 and/or 2 or variants thereof.

8. An expression vector comprising the nucleic acid sequence of claims 6 or 7 and one or more regulatory elements.

9. The expression vector of claim 8, wherein the regulatory elements contain a promoter selected from constitutive or inducible promoters.

10. The expression vector of claim 9, wherein the promoters are selected from GPD, GAL4, CUP1, MET25, GAL1 or GAL1-10.

11. The expression vector of claims 8-10, which is a plasmid.

12. An expression vector comprising a nucleic acid coding for prolyl-4-hydroxylase, wherein the nucleic acid is linked to a signal sequence for efficient transport of said sequence to the ER.

13. The expression vector of claim 12, wherein the signal sequence is a host, preferably yeast, specific signal sequence, preferably mating factor alpha 1 (MFa) of *S. cerevisiae.*

14. A kit of parts or a co-expression system comprising the following constituents:
a) the expression vector of one or more of claims 8-11; and
b) an expression vector of claim 12 or 13;

15. A host cell, which has been transformed with the expression vectors of one or more of claims 8-13 or with the co-expression system of claim 14.

16. The host cell of claim 15, which is a eukaryotic cell.

17. The host cell of claim 16, which is a mammalian cell, plant cell, yeast cell or an insect cell.

18. The host cell of claim 17, which is a yeast cell, preferably a *S. cerevisiae, Schizosaccharomyces pombe, Pichia pastoris, Candida albicans,* or *Hansenula polymorpha* cell.

19. The host cell of claim 17, which is a mammalian cell selected from a CHO, COS, HeLa, 293T, HEH or BHK cell.

20. The host cell of claim 17, which is an insect cell selected from Lepidoptera insect cells, preferably from Spodoptera frugiperda and from Trichoplusia ni.

21. The host cell of claim 20, wherein the insect cell is a Sf9, Sf21 or high five cell.

22. The host cell of claim 17, wherein the plant cell is derived from tobacco, potato, corn, pea and tomato.

23. A method of producing recombinant mussel byssus proteins comprising the steps of:
a) providing a host cell of one of claims 15-22;
b) transforming said host cell with a vector of one or more of claims 8-13 or the co-expression system of claim 14;
c) expressing recombinant mussel byssus protein from said host cell under suitable conditions; and
d) recovering said mussel byssus protein.

24. A method for producing threads from recombinant mussel byssus protein, comprising the following steps:
a) providing recombinant mussel byssus protein produced in claim 23, and
b) (electro)spinning or moulding said protein into threads by a suitable method.

25. A thread obtainable by the method of claim 24.

26. Use of the proteins/threads of claims 1-5 or 25 in the field of biotechnology and/or medicine.

27. Use of the proteins/threads of claims 1-5 or 25 for the manufacture of wound closure or coverage systems.

28. The use of claim 27 for the manufacture of suture materials.

29. The use of claim 28, wherein the suture material is intended for use in neurosurgery or ophthalmic surgery.

30. Use of the proteins/threads of claims 1-5 or 25 for the manufacture of replacement materials, preferably artificial cartilage or tendon materials.

31. Wound closure or coverage systems, suture materials, replacement materials, preferably artificial cartilage or tendon materials, which are obtainable using proteins/threads of claims 1-5 or 25.

32. Cosmetics, drug delivery vehicles, fabrics, textile, paper product, leather product, automotive parts or aircraft parts, which contain proteins/threads of claims 1-5 or 25.
